# EUROPÄISCHE PATENTSCHRIFT

(11) **EP 2 606 034 B1**
(45) Veröffentlichungstag und Bekanntmachung des Hinweises auf die Patenterteilung: **09.03.2016**
(21) Anmeldenummer: 11743438.1
(22) Anmeldetag: 19.07.2011
(51) Int. Cl.: C07D 233/88, C07D 401/04, C07D 401/12, C07D 401/14, C07D 403/14, C07D 413/14, A61K 31/505, A61P 35/00

(54) **PYRIMIDINDERIVATE ALS FAK INHIBITOREN**
PYRIMIDINE DERIVATIVE AS FAK INHIBITORS
DERIVATES DE PYRIMIDINE COMME INHIBITEURS DE FAK

(30) Priorität: 18.08.2010 DE 102010034699
(43) Veröffentlichungstag der Anmeldung: 26.06.2013
(73) Patentinhaber: Merck Patent GmbH, 64293 Darmstadt (DE)
(72) Erfinder: HEINRICH, Timo, 64823 Gross-Umstadt (DE); ESDAR, Christina, 55128 Mainz (DE); GREINER, Hartmut, 64331 Weiterstadt (DE)
(86) Internationale Anmeldenummer: PCT/EP2011/003597
(87) Internationale Veröffentlichungsnummer: WO 2012/022408

(56) Entgegenhaltungen:
- WO-A1-2004/056807
- WO-A1-2006/110763
- WO-A2-03/087087
- BENOÎT BLANK ET AL: "Catalytic Alkylation of Methyl- N -Heteroaromatics with Alcohols", JOURNAL OF THE AMERICAN CHEMICAL SOCIETY, Bd. 132, Nr. 3, 27. Januar 2010 (2010-01-27), Seiten 924-925, XP55006344, ISSN: 0002-7863, DOI: 10.1021/ja9095413
- MATSUKAWA TAIZO ET AL: "Reactions of 2-aminopyrimidines with aromatic aldehydes. I", YAKUGAKU ZASSHI - JOURNAL OF THE PHARMACEUTICAL SOCIETY OF JAPAN, JAPAN SCIENCE AND TECHNOLOGY INFORMATION AGGREGATOR, ELECTRONIC, JP, Bd. 72, Nr. 7, 1. Januar 1952 (1952-01-01) , Seiten 909-912, XP008142564, ISSN: 0031-6903
- ALEXANDER SCHULTZE & WALTER FIEDLER: "Therapeutic potential and limitations of new FAK inhibitors in the treatment of cancer", EXPERT OPINION ON INVESTIGATIONAL DRUGS, ASHLEY PUBLICATIONS LTD., LONDON, GB, Bd. 19, Nr. 6, 1. Juni 2010 (2010-06-01), Seiten 777-788, XP008142458, ISSN: 1354-3784, DOI: 10.1517/13543784.2010.489548

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation der Signaltransduktion von Kinasen, insbesondere der Tyrosinkinasen eine Rolle spielt, ferner pharmazeutische Zusammensetzungen, die diese Verbindungen enthalten, sowie die Verwendung der Verbindungen zur Behandlung von kinasebedingter Krankheiten.

Im einzelnen betrifft die vorliegende Erfindung Verbindungen, die die Signaltransduktion der Tyrosinkinasen hemmen, regulieren und/oder modulieren, Zusammensetzungen, die diese Verbindungen enthalten, sowie Verfahren zu ihrer Verwendung zur Behandlung von tyrosinkinasebedingten Krankheiten und Leiden wie Krebs, Tumorwachstum, Arteriosklerose, altersbedingte Makula-Degeneration, diabetische Retinopathie, Entzündungserkrankungen und dergleichen bei Säugetieren.
Bei den Tyrosinkinasen handelt es sich um eine Klasse von Enyzmen, die die Übertragung des endständigen Phosphats des Adenosintriphosphats auf Tyrosinreste bei Proteinsubstraten katalysieren. Man nimmt an, dass den Tyrosinkinasen bei verschiedenen Zellfunktionen über die Substratphosphorylierung eine wesentliche Rolle bei der Signaltransduktion zukommt. Obwohl die genaue Mechanismen der Signaltransduktion noch unklar sind, wurde gezeigt, dass die Tyrosinkinasen wichtige Faktoren bei der Zellproliferation, der Karzinogenese und der Zelldifferenzierung darstellen.

Die Tyrosinkinasen lassen sich in Rezeptor-Tyrosinkinasen und zytosolische Tyrosinkinasen einteilen. Die Rezeptor-Tyrosinkinasen weisen einen extrazellulären Teil, einen Transmembranteil und einen intrazellulären Teil auf, während die zytosolischen Tyrosinkinasen ausschließlich intrazellulär vorliegen.
Die zytosolischen Tyrosinkinasen bestehen aus einer Vielzahl von Unterfamilien, darunter Src, Frk, Btk, Csk, Abl, Zap70, Fes/Fps, Fak, Jak, Ack, and LIMK. Jede dieser Unterfamilien ist weiter in verschiedene Rezeptoren unterteilt. Für eine genauere Diskussion der zytosolischen Tyrosinkinasen, siehe die Arbeit von Bolen Oncogene, 8:2025-2031 (1993), die hiermit durch Bezugnahme aufgenommen wird.
Sowohl die Rezeptor-Tyrosinkinasen als auch die zytosolischen Tyrosinkinasen sind an Signalübertragungswegen der Zelle, die zu verschiedenen Leidenszuständen führen, darunter Krebs, Schuppenflechte und Hyperimmunreaktionen, beteiligt.

Die vorliegende Erfindung betrifft die Verbindungen als Inhibitoren von FAK (Focal Adhesion Kinase).
FAK (die durch das Gen PTK2 kodiert wird) ist eine Nicht-Rezeptor-TyrosinKinase, die Signale von Integrinen und Wachstumsfaktor-Rezeptoren integriert. Es wurde berichtet, dass FAK eine Rolle bei der Regulierung des Überlebens, des Wachstums, der Verbreitung, Migration sowie Invasion von Zellen spielt (McLean et al. 2005, Nat Rev Cancer 5:505-515). Darüber hinaus wird FAK mittels Phosphorylierung an mehreren Tyrosinresten reguliert und aktiviert. Bei vielen humanen Tumoren, einschließlich Brustkrebs, Darmkrebs, Schilddrüsenkrebs und Prostatakrebs wurde eine Überexpression von FAK-mRNA und/oder Protein dokumentiert (Owens et al. 1995, Cancer Research 55: 2752-2755; Agochiya et al. 1999, Oncogene 18: 5646-5653; Gabarro-Niecko et al. 2003, Cancer Metastasis Rev. 22: 359-374). Noch wichtiger sind Hinweise, nach denen phosphorylierte FAK im Vergleich zu normalen Geweben in malignen Geweben erhöht ist (Grisaru-Granovsky et al. 2005, Int. J. Cancer 113: 372-378).

Die Inhibierung von FAK durch RNAi oder Expression eines dominantnegativen FAK induzierte nachweislich einen Adhäsionsverlust und Zelltod bei humanen Brust- und Melanomzelllinien und erhöhte Docetaxel-vermittelte Apoptose bei Eierstockkrebszellen (Beviglia et al 2003, Biochem J. 373:201-210, Smith et al 2005, Melanoma Res. 15: 357-362, Haider et al 2005, Clin. Cancer Res. 11: 8829-8836). Die Inhibierung von FAK bei normalen humanen Fibroblasten oder immortalisierten Säugetierzellen (MCFIOA) erwies sich jedoch nicht als Auslöser für den Bindungsverlust oder Apoptose (Xu et al. 1996 Cell Growth and Diff 7: 413-418). Auch die Inhibierung von FAK durch dominant-negative Expression verminderte nachweislich Tumorwachstum und eliminierte in einem syngenetischen Rattenmodell Lungenmetastasen bei Säugetier-Adenokarzinomzellen (van Nimwegen et al 2005, Cancer Res. 65: 4698-4706). Ebenso inhibierte die Inhibierung von FAK durch shRNA Lungenmetastasen und verringerte die Letalität in einem syngenetischen Mausmodell um 40 % (Mitra et al 2006, Oncogene 25: 4429-4440). In dieser Untersuchung führte transiente Reexpression der Wildtyp-, aber nicht kinaseinaktiven FAK zur Rückmutation des shRNA-Phenotyps. Inhibierung von FAK durch dominant-negative Expression in Maus-4TI-Karzinomzellen verringerte Tumorwachstum und Angiogenese bei Mäusen (Mitra et al 2006, Oncogene 25:5969-5984).

Darüber hinaus verringerte der Verlust an katalytischer Aktivität von FAK (Rekonstituierung von FAK-/-Zellen mit kinase-inaktiver FAK) das Wachstum von v-Src-Tumoren bei Mäusen und reduzierte Angiogenese.

Daher existieren starke Anhaltspunkte, die darauf hinweisen, dass die Inhibierung der FAK-Aktivität Apoptose, Verlust von Adhäsion, Inhibierung des Zellwachstums und Migration induziert und dass solch eine Inhibierung Angiogenese verringert. Demzufolge wären Verbindungen, welche die FAK-Aktivität inhibieren, für die Behandlung von Krebs von Nutzen.

Die Identifikation von kleinen Verbindungen, die die Signaltransduktion der FAK spezifisch hemmen, regulieren und/oder modulieren, ist daher wünschenswert und ein Ziel der vorliegenden Erfindung.

Es wurde gefunden, daß die Verbindungen gemäß Anspruch 1 und ihre Salze bei guter Verträglichkeit sehr wertvolle pharmakologische Eigenschaften besitzen.

Insbesondere zeigen sie inhibierende Eigenschaften der FAK.
Ein weiterer Gegenstand der vorliegenden Erfindung ist die Verwendung einer oder mehrerer erfindungsgemäßer Verbindungen bei der Behandlung und/oder Prophylaxe von Erkrankungen, bevorzugt den hier beschriebenen Erkrankungen, die durch FAK verursacht, vermittelt und/oder propagiert werden.
Gewöhnlich werden die hier besprochenen Erkrankungen in zwei Gruppen eingeteilt, in hyperproliferative und nicht hyperproliferative Erkrankungen. In diesem Zusammenhang werden Psoriasis, Arthritis, Entzündungen, Endometriose, Vernarbung, gutartige Prostatahyperplasie, immunologische Krankheiten, Autoimmunkrankheiten und Immunschwächekrankheiten als nicht krebsartige Krankheiten angesehen, von denen Arthritis, Entzündung, immunologische Krankheiten, Autoimmunkrankheiten und Immunschwächekrankheiten gewöhnlich als nicht hyperproliferative Erkrankungen angesehen werden. In diesem Zusammenhang sind Hirnkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie als krebsartige Erkrankungen anzusehen, die alle gewöhnlich als hyperproliferative Erkrankungen angesehen werden. Insbesondere krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.

Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer-(HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J., unmittelbar vor der Veröffentlichung, Manuskript BJ20020786).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### STAND DER TECHNIK

Pyridinderivate sind als FAK-Inhibitoren in der WO 2009/105498 und in der WO 2008/115369 beschrieben.

Andere Pyrimidinderivate zur Krebsbekämpfung kennt man aus WO 2004/056807 A1 und von A. Schultze et al., Expert Opinion Invest. Drugs, Bd. 19, Nr. 6, 2010, 777-788, XP008142458, sowie aus WO 2010/055117.

### ZUSAMMENFASSUNG DER ERFINDUNG

Die Erfindung betrifft einzelne Verbindungen gemäß Anspruch 1 umfaßt von der Formel I 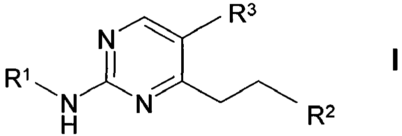 worin
- R¹: (CH₂)ₙAr¹ oder (CH₂)ₙHet¹,
- R²: Ar² oder Het²,
- R³: H, Hal, A, Het⁴ oder CN,
- Ar¹: unsubstituiertes oder ein-, zwei-, drei-, vier oder fünffach durch Hal, A, (CH₂)ₙOH, (CH₂)ₙOA, (CH₂)ₙCN, NO₂, SO₂A, COOH, COOA, NH₂, NHA, NA₂, NHCH₂Ar¹, CHO, COA, (CH₂)ₙCONH₂, (CH₂)ₙCONHA, (CH₂)ₙCONA₂, Het³, NHCOHet³, SO₂NH₂, SO₂NHA und/oder NHCOA substituiertes Phenyl,
- Ar²: unsubstituiertes oder ein-, zwei-, drei-, vier oder fünffach durch Hal, A, (CH₂)ₙOH, (CH₂)ₙOA, (CH₂)ₙCN, NO₂, SO₂A, COOH, COOA, NH₂, NHA, NA₂, NHCH₂Ar¹, CHO, COA, (CH₂)ₙCONH₂, (CH₂)ₙCONHA, (CH₂)ₙCONA₂, SO₂NH₂, NHSO₂A, SO₂NHA, NA'SO₂A, C(R⁴R⁵)CN und/oder NHCOA substituiertes Phenyl,
- Het¹: einen ein- oder zweikernigen aromatischen, ungesättigten oder gesättigten Heterocyclus mit 1 bis 4 N-, O- und/oder S-Atomen, der unsubstituiert oder ein-, zwei-, drei- oder vierfach durch Hal, A, NH₂, NHA, NA₂, COOH, CN, NHCOA, COA, OAr, COOA, CONH₂, CONHA, CONA₂, CONHAr und/oder =O substituiert sein kann,
- Het²: einen ein- oder zweikernigen aromatischen Heterocyclus mit 1 bis 4 N-, O- und/oder S-Atomen, der unsubstituiert oder ein-, zwei-, drei- oder vierfach durch Hal, A, NH₂, NHA, NA₂, COOH, COA, COOA, CONH₂, CONHA, CONA₂, CONHAr, C(R⁴R⁵)CN, NHSO₂A, NASO₂A, NHCOA, NA'COA, NACHO, NH(CH₂)ₚNHCHO und/oder =O substituiert sein kann,
- Ar: unsubstituiertes oder ein-, zwei- oder dreifach durch Hal und/oder A substituiertes Phenyl,
- R⁴, R⁵: jeweils unabhängig voneinander H oder A,
- R⁴ und R⁵: zusammen auch Alkylen mit 2-5 C-Atomen, worin eine CH₂-Gruppe durch NH, NA', O oder S ersetzt sein kann,
- Het³: einen ein- oder zweikernigen aromatischen, ungesättigten oder gesättigten Heterocyclus mit 1 bis 3 N-, O- und/oder S-Atomen, der unsubstituiert oder ein- oder zweifach durch A, NH₂ und/oder =O substituiert sein kann,
- Het⁴: einen einkernigen aromatischen Heterocyclus mit 1 bis 3 N-, O- und/oder S-Atomen, der unsubstituiert oder ein- oder zweifach durch A substituiert sein kann,
- A: unverzweigtes oder verzweigtes Alkyl mit 1-10 C-Atomen, worin 1-7 H-Atome durch F ersetzt sein können und/oder worin eine oder zwei nicht-benachbarte CH- und/oder CH₂-Gruppen durch O, NH, NA', S, SO, SO₂ und/oder durch CH=CH-Gruppen ersetzt sein können,
oder
cyclisches Alkyl mit 3-7 C-Atomen,
- A': unverzweigtes oder verzweigtes Alkyl mit 1-6 C-Atomen,
- Hal: F, Cl, Br oder I,
- n: 0, 1, 2, 3 oder 4,
- p: 1, 2, 3 oder 4
bedeuten,
sowie ihre pharmazeutisch verwendbaren Salze, Tautomere und Stereoisomere, einschließlich deren Mischungen in allen Verhältnissen.

Der Anmeldungsgegenstand bezieht sich auf die Definition der Ansprüche; jede Offenbarung, die über den Umfang der Ansprüche hinausgeht, dient nur zu Informationszwecken.

Gegenstand der Erfindung sind die Verbindungen der Formel I gemäß Anspruch 1 und ihre Salze sowie ein Verfahren zur Herstellung von Verbindungen der Formel I sowie ihrer pharmazeutisch verwendbaren Salze und Stereoisomere, dadurch gekennzeichnet, daß man
a) eine Verbindung der Formel II 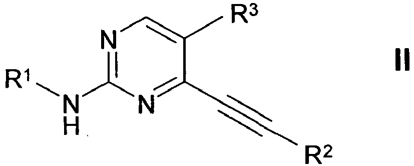 worin
   R¹, R² und R³ die in Anspruch 1 entsprechenden Bedeutungen haben,
   hydriert,
   oder
b) eine Verbindung der Formel III 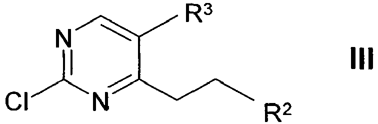 worin
   R² und R³ die in Anspruch 1 entsprechenden Bedeutungen haben,
   mit einer Verbindung der Formel IV

      R¹-NH₂ IV

      worin R¹ die in Anspruch 1 entsprechende Bedeutung hat,
      umsetzt,
   und/oder
   eine Base oder Säure der Formel I in eines ihrer Salze umwandelt.

Unter Verbindungen der Formel I gemäß Anspruch 1 versteht man auch die Hydrate und Solvate dieser Verbindungen.
Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Unter pharmazeutisch verwendbaren Derivaten versteht man z.B. die Salze der erfindungsgemäßen Verbindungen.

Gegenstand der Erfindung sind auch Mischungen der erfindungsgemäßen Verbindungen, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.

Für alle Reste, die mehrfach auftreten, wie z.B. A, gilt, daß deren Bedeutungen unabhängig voneinander sind.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2-, 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

A' bedeutet vorzugsweise Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl oder Benzyl.

OA bedeutet Alkoxy und ist vorzugsweise z.B. Methoxy, Ethoxy, Propoxy, Isopropoxy, Butoxy, Trifluormethoxy oder Cyclopentoxy.

-COA (Acyl) bedeutet vorzugsweise Acetyl, Propionyl, ferner auch Butyryl, Pentanoyl, Hexanoyl oder z.B. Benzoyl. Hal bedeutet vorzugsweise F, Cl oder Br, aber auch I.

R³ bedeutet H, Hal, A, Het⁴ oder CN, besonders bevorzugt H, Hal, A oder CN, ferner auch Het⁴.

Ar¹ bedeutet z.B. unsubstituiertes Phenyl, weiterhin vorzugsweise z.B. durch A, Fluor, Chlor, Brom, Iod, Hydroxy, Methoxy, Ethoxy, Propoxy, Butoxy, Pentyloxy, Hexyloxy, Nitro, Cyan, Formyl, Acetyl, Propionyl, Trifluormethyl, Amino, Methylamino, Ethylamino, Dimethylamino, Diethylamino, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl, Het³ und/oder NHCOHet³ mono-, di- oder trisubstituiertes Phenyl.
Ar¹ bedeutet besonders bevorzugt ein-, zwei-, drei-, vier oder fünffach durch Hal, (CH₂)nCN, SO₂A, (CH₂)ₙCONH₂, (CH₂)ₙCONHA, (CH₂)ₙCONA₂, Het³ und/oder NHCOHet³ substituiertes Phenyl.

Ar² bedeutet z.B. unsubstituiertes Phenyl, weiterhin vorzugsweise z.B. durch A, Fluor, Chlor, Brom, Iod, Hydroxy, Methoxy, Ethoxy, Propoxy, Butoxy, Pentyloxy, Hexyloxy, Nitro, Cyan, Formyl, Acetyl, Propionyl, Trifluormethyl, Amino, Methylamino, Ethylamino, Dimethylamino, Diethylamino, Sulfonamido, Methylsulfonamido, Ethylsulfonamido, Propylsulfonamido, Butylsulfonamido, Dimethylsulfonamido, Phenylsulfonamido, Carboxy, Methoxycarbonyl, Ethoxycarbonyl, Aminocarbonyl und/oder C(R⁴R⁵)CN mono-, di- oder trisubstituiertes Phenyl.

Ar² bedeutet besonders bevorzugt ein-, zwei-, drei-, vier oder fünffach durch Hal, A, (CH₂)ₙCN, SO₂A, NHSO₂A, NA'SO₂A und/oder C(R⁴R⁵)CN substituiertes Phenyl.

Het¹ bedeutet, von den möglichen Substituenten abgesehen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder-5-yl, 1,3,4-Thiadiazol-2- oder-5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Chromenyl.
Die heterocyclischen Reste können auch teilweise oder vollständig hydriert sein.
Het¹ kann also z. B. auch bedeuten 2,3-Dihydro-2-, -3-, -4- oder -5-furyl, 2,5-Dihydro-2-, -3-, -4- oder 5-furyl, Tetrahydro-2- oder-3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder-4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder-6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder-5-yl, Hexahydro-1-, -3- oder-4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder-8-chinolyl), 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethyfendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydro-benzofuranyl oder 2,3-Dihydro-2-oxo-furanyl.

Het¹ bedeutet besonders bevorzugt unsubstituiertes oder ein-, zwei- oder dreifach durch A, CN, NHCOA, COA, OAr und/oder =O substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl, Tetrahydrochinolyl, Dihydro-benzoxazolyl, Dihydropyridazinyl, Dihydrobenzimidazolyl, Dihydrobenzothiazolyl, Piperidinyl, Pyrrolidinyl, Morpholinyl, Piperazinyl, Imidazolidinyl, Oxazolidinyl oder Tetrahydropyranyl.

Het² bedeutet vorzugsweise ein-, zwei- oder dreifach durch C(R⁴R⁵)CN, NHSO₂A, NASO₂A, NHCOA, NA'COA, NACHO, NH(CH₂)ₚNHCHO und/oder =O substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl.

Het³ bedeutet vorzugsweise unsubstituiertes oder ein-, zwei- oder dreifach durch A, NH₂ und/oder =O substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl, Pyrazinyl, Benzimidazolyl, Benzotriazolyl, Chinolinyl, Chinoxalinyl, Chinazolinyl, Pyrrolopyridinyl, Purinyl, Indolyl oder Indazolyl, Tetrahydrochinolyl, Dihydrobenzoxazolyl, Dihydropyridazinyl, Dihydrobenzimidazolyl, Dihydrobenzothiazolyl, Piperidinyl, Pyrrolidinyl, Morpholinyl, Piperazinyl, Imidazolidinyl, Oxazolidinyl oder Tetrahydropyranyl.

Het⁴ bedeutet einen einkernigen aromatischen Heterocyclus mit 1 bis 3 N-, O- und/oder S-Atomen, der unsubstituiert oder ein- oder zweifach durch A substituiert sein kann.
Het⁴ bedeutet vorzugsweise unsubstituiertes oder ein- oder zweifach durch A, substituiertes Furyl, Thienyl, Pyrrolyl, Imidazolyl, Pyrazolyl, Oxazolyl, Isoxazolyl, Thiazolyl, Isothiazolyl, Pyridyl, Pyrimidinyl, Triazolyl, Tetrazolyl, Oxadiazolyl, Thiadiazolyl, Pyridazinyl oder Pyrazinyl.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Ausgangsverbindungen der Formeln II, III und IV sind in der Regel bekannt. Sind sie neu, so können sie aber nach an sich bekannten Methoden hergestellt werden.

Verbindungen der Formel I können vorzugsweise erhalten werden, indem man Verbindungen der Formel II hydriert, vorzugsweise mit Wasserstoff. Die Umsetzung erfolgt nach Methoden, die dem Fachmann bekannt sind. Die Reaktion erfolgt in einem inerten Lösungsmittel, vorzugsweise Methanol.
Als Katalysator dient vorzugsweise Palladium auf Aktivkohle. Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

Verbindungen der Formel I können weiterhin vorzugsweise erhalten werden, indem man Verbindungen der Formel III mit Verbindungen der Formel IV umsetzt.
Die Umsetzung erfolgt in einem inerten Lösungsmittel.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist n-Butanol.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der erfindungsgemäßen Verbindungen werden größtenteils konventionell hergestellt. Sofern die erfindungsgemäße Verbindung eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I gemäß Anspruch 1 zählen ebenfalls dazu. Bei bestimmten Verbindungen der Formel I gemäß Anspruch 1 lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I gemäß Anspruch 1 die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Palmoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der erfindungsgemäßen Verbindungen, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer erfindungsgemäßer Verbindungen werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der erfindungsgemäßen Verbindungen mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine erfindungsgemäße Verbindung in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nicht-chemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine erfindungsgemäße Verbindung und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,1 mg bis 3 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nicht-toxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der vorliegenden Erfindung hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel I gemäß Anspruch 1 und/oder ihre physiologisch unbedenklichen Salze zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.
Ebensfalls umfasst ist die Verwendung der erfindungsgemäßen Verbindungen nach Anspruch 1 und/oder ihre physiologisch unbedenklichen Salze zur Herstellung eines Arzneimittels zur Behandlung oder Vorbeugung einer Krankheit, an der Angiogenese beteiligt ist.
Eine derartige Krankheit, an der Angiogenese beteiligt ist, ist eine Augenkrankheit, wie Retina-Vaskularisierung, diabetische Retinopathie, altersbedingte Makula-Degeneration und dergleichen.

Die Verwendung von Verbindungen der Formel I gemäß Anspruch 1 und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung der Verbindungen der Formel I gemäß Anspruch 1 und/oder ihre physiologisch unbedenklichen Salze zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.
Die vorliegende Erfindung umfasst auch die Verwendung Verbindungen der Formel I gemäß Anspruch 1 und/oder ihre physiologisch unbedenklichen Salze und Solvate zur Herstellung eines Arzneimittels zur Behandlung oder Vorbeugung von Retina-Vaskularisierung.
Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.
Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel I gemäß Anspruch 1 können an Patienten zur Behandlung von Krebs, insbesondere schnell wachsenden Tumoren, verabreicht werden.

Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren der Lunge, des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens und/oder des Kehlkopfs.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die offenbarten Verbindungen der Formel I gemäß Anspruch 1 können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD-1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (Cl-1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-ανβ3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS-2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und Invivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I gemäß Anspruch 1 kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | BBR-3464 (Hoffmann-La Roche) |
| | Ormiplatin | SM-11355 (Sumitomo) |
| | Iproplatin | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | Ethinylcytidin (Taiho) |
| | | |
| Topoisomerase-Inhibitoren | Amsacrin | Rubitecan (SuperGen) |
| | Epirubicin | Exatecanmesylat (Daiichi) |
| | Etoposid | Quinamed (ChemGenex) |
| | Teniposid oder Mitoxantron | Gimatecan (Sigma- Tau) |
| | Irinotecan (CPT-11) | Diflomotecan (Beaufour-Ipsen) |
| | 7-Ethyl-10-hydroxycamptothecin | TAS-103 (Taiho) |
| | Topotecan | Elsamitrucin (Spectrum) |
| | Dexrazoxanet (TopoTarget) | J-107088 (Merck & Co) |
| | Pixantron (Novuspharrna) | BNP-1350 (BioNumerik) |
| | Rebeccamycin-Analogon (Exelixis) | CKD-602 (Chong Kun Dang) |
| | | KW-2170 (Kyowa Hakko) |
| | BBR-3576 (Novuspharrna) | |
| | | |
| Antitumor-Antibiotika | Dactinomycin (Actinomycin D) | Amonafid |
| | Doxorubicin (Adriamycin) | Azonafid |
| | Deoxyrubicin | Anthrapyrazol |
| | Valrubicin | Oxantrazol |
| | Daunorubicin (Daunomycin) | Losoxantron |
| | Epirubicin | Bleomycinsulfat (Blenoxan) |
| | Therarubicin | Bleomycinsäure |
| | Idarubicin | Bleomycin A |
| | Rubidazon | Bleomycin B |
| | Plicamycinp | Mitomycin C |
| | Porfiromycin | MEN-10755 (Menarini) |
| | Cyanomorpholinodoxorubicin | GPX-100 (Gem |
| | Mitoxantron (Novantron) | Pharmaceuticals) |
| | | |
| Antimitotische Mittel | Paclitaxel | SB 408075 (GlaxoSmithKline) |
| | Docetaxel | E7010 (Abbott) |
| | Colchicin | PG-TXL (Cell Therapeutics) |
| | Vinblastin | IDN 5109 (Bayer) |
| | Vincristin | A 105972 (Abbott) |
| | Vinorelbin | A 204197 (Abbott) |
| | Vindesin | LU 223651 (BASF) |
| | Dolastatin 10 (NCI) | D 24851 (ASTA Medica) |
| | Rhizoxin (Fujisawa) | ER-86526 (Eisai) |
| | Mivobulin (Warner-Lambert) | Combretastatin A4 (BMS) |
| | Cemadotin (BASF) | Isohomohalichondrin-B (PharmaMar) |
| | RPR 109881A (Aventis) | |
| | TXD 258 (Aventis) | ZD 6126 (AstraZeneca) |
| | Epothilon B (Novartis) | PEG-Paclitaxel (Enzon) |
| | T 900607 (Tularik) | AZ10992 (Asahi) |
| | T 138067 (Tularik) | !DN-5109 (Indena) |
| | Cryptophycin 52 (Eli Lilly) | AVLB (Prescient NeuroPharma) |
| | Vinflunin (Fabre) | Azaepothilon B (BMS) |
| | Auristatin PE (Teikoku Hormone) | BNP- 7787 (BioNumerik) |
| | BMS 247550 (BMS) | CA-4-Prodrug (OXiGENE) |
| | BMS 184476 (BMS) | Dolastatin-10 (NrH) |
| | BMS 188797 (BMS) | CA-4 (OXiGENE) |
| | Taxoprexin (protarga) | |
| | | |
| Aromatase-Inhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylat-synthase-Inhibitoren | Pemetrexed (Eli Lilly) | Nolatrexed (Eximias) |
| | ZD-9331 (BTG) | CoFactor™ (BioKeys) |
| | | |
| DNA-Antagonisten | Trabectedin (PharmaMar) | Mafosfamid (Baxter International |
| | Glufosfamid (Baxter International) | Apaziquon (Spectrum Pharmaceuticals) |
| | Albumin + 32P (Isotope Solutions) | |
| | | 06-Benzylguanin (Paligent) |
| | Thymectacin (NewBiotics) | |
| | Edotreotid (Novartis) | |
| | | |
| Farnesyltransferase-Inhibitoren | Arglabin (NuOncology Labs) | Tipifarnib (Johnson & Johnson) |
| | lonafarnib (Schering-Plough) | Perillylalkohol (DOR BioPharma) |
| | BAY-43-9006 (Bayer) | |
| | | |
| Pumpen-Inhibitoren | CBT-1 (CBA Pharma) | Zosuquidar-Trihydrochlorid (Eli L |
| | Tariquidar (Xenova) | Biricodar-Dicitrat (Vertex) |
| | MS-209 (Schering AG) | |
| | | |
| Histonacetyltransfera Inhibitoren | Tacedinalin (Pfizer) | Pivaloyloxymethylbutyrat (Titan) |
| | SAHA (Aton Pharma) | Depsipeptid (Fujisawa) |
| | MS-275 (Schering AG) | |
| | | |
| Metalloproteinase-Inhibitoren | Neovastat (Aeterna Laboratories) | CMT -3 (CollaGenex) |
| | Marimastat (British Biotech) | BMS-275291 (Celltech) |
| Ribonucleosid-reduktase-Inhibitoren | Galliummaltolat (Titan) | Tezacitabin (Aventis) |
| | Triapin (Vion) | Didox (Molecules for Health) |
| | | |
| TNF-alpha-Agonisten / Antagonis | Virulizin (Lorus Therapeutics) | Revimid (Celgene) |
| | CDC-394 (Celgene) | |
| | | |
| Endothelin-A-Rezeptc Antagonisten | Atrasentan (Abbot) | YM-598 (Yamanouchi) |
| | ZD-4054 (AstraZeneca) | |
| | | |
| Retinsäure-rezeptor-Agonisten | Fenretinid (Johnson & Johnson) | Alitretinoin (Ligand) |
| | LGD-1550 (Ligand) | |
| | | |
| Immunmodulatoren | Interferon | Dexosom-Therapie (Anosys) |
| | Oncophaqe (Antiqenics) | Pentrix (Australian Cancer |
| | GMK (Progenics) | Technology) |
| | Adenokarzinom-I mpfstoff (Biomira) | JSF-154 (Tragen) |
| | | Krebsimpfstoff (Intercell) |
| | CTP-37 (AVI BioPharma) | Norelin (Biostar) |
| | JRX-2 (Immuno-Rx) | BLP-25 (Biomira) |
| | PEP-005 (Peplin Biotech) | MGV (Progenics) |
| | Synchrovax-Impfstoffe (CTL Immuno) | !3-Alethin (Dovetail) |
| | Melanom-Impfstoff (CTL Immuno) | CLL-Thera (Vasogen) |
| | | |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteroncaproat | Goserelin |
| | Medroxyprogesteron | Leuporelin |
| | Testosteron | Bicalutamid |
| | Testosteronpropionat | Flutamid |
| | Fluoxymesteron | Octreotid |
| | Methyltestosteron | Nilutamid |
| | Diethylstilbestrol | Mitotan |
| | Megestrol | P-04 (Novogen) |
| | Tamoxifen | 2-Methoxyöstradiol (EntreMed) |
| | Toremofin | Arzoxifen (Eli Lilly) |
| | Dexamethason | |
| | | |
| Photodynamische Mittel | Talaporfin (Light Sciences) | Pd-Bacteriopheophorbid (Yeda) |
| | Theralux (Theratechnologies) | Lutetium-Texaphyrin (Pharmacyclics) |
| | Motexafin-Gadolinium (Pharmacyclics) | |
| | | Hypericin |
| | | |
| Tyrosinkinase-Inhibitoren | Imatinib (Novartis) | Kahalid F (PharmaMar) |
| | Leflunomid (Sugen/Pharmacia) | CEP- 701 (Cephalon) |
| | ZDI839 (AstraZeneca) | CEP-751 (Cephalon) |
| | Erlotinib (Oncogene Science) | MLN518 (Millenium) |
| | Canertjnib (Pfizer) | PKC412 (Novartis) |
| | Squalamin (Genaera) | Phenoxodiol O |
| | SU5416 (Pharmacia) | Trastuzumab (Genentech) |
| | SU6668 (Pharmacia) | C225 (ImClone) |
| | ZD4190 (AstraZeneca) | rhu-Mab (Genentech) |
| | ZD6474 (AstraZeneca) | MDX-H210 (Medarex) |
| | Vatalanib (Novartis) | 2C4 (Genentech) |
| | PKI166 (Novartis) | MDX-447 (Medarex) |
| | GW2016 (GlaxoSmithKline) | ABX-EGF (Abgenix) |
| | EKB-509 (Wyeth) | IMC-1C11 (ImClone) |
| | EKB-569 (Wyeth) | |
| Verschiedene Mittel | SR-27897 (CCK-A-Inhibitor, Sanofi-Synthelabo) | BCX-1777 (PNP-Inhibitor, BioCryst) |
| | | |
| | Tocladesin (cyclisches-AMP-Agonist, Ribapharm) | Ranpirnase (Ribonuclease-Stimulans, Alfacell) |
| | | |
| | Alvocidib (CDK-Inhibitor, Aventis) | Galarubicin (RNA-Synthese-Inhibitor, Dong-A) |
| | CV-247 (COX-2-Inhibitor, Ivy Medical) | |
| | P54 (COX-2-Inhibitor, Phytopharm) | Tirapazamin (Reduktionsmittel, SRI International) |
| | | |
| | CapCell™ (CYP450-Stimulans, Bavarian Nordic) | N-Acetylcystein (Reduktions-mittel, Zambon) |
| | GCS-IOO (gal3-Antagonist, GlycoGenesys) | R-Flurbiprofen (NF-kappaB-Inhibitor, Encore) |
| | G17DT-Immunogen (Gastrin-Inhibitor Aphton) | 3CPA (NF-kappaB-Inhibitor, Active Biotech) |
| | Efaproxiral (Oxygenator, Allos Therapeutics) | Seocalcitol (Vitamin-D-Rezeptor-Agonist, Leo) |
| | PI-88 (Heparanase-Inhibitor, Progen) | 131-I-TM-601 (DNA-Antagonist, TransMolecular) |
| | Tesmilifen (Histamin-Antagonist, YM BioSciences) | Eflornithin (ODC-Inhibitor, ILEX Oncology) |
| | Histamin (Histamin-H2-Rezeptor-Agonist, Maxim) | Minodronsäure (Osteoclasten-Inhibitor, Yamanouchi) |
| | Tiazofurin (IMPDH-Inhibitor, Ribapharm) | Indisulam (p53-Stimulans, Eisai) |
| | | Aplidin (PPT-Inhibitor, PharmaMar) |
| | Cilengitid (Integrin-Antagonist, Merck KGaA) | |
| | | Rituximab (CD20-Antikörper, Genentech) |
| | SR-31747 (IL-1-Antagonist, Sanofi-Synthelabo) | |
| | | Gemtuzumab (CD33-Antikörper, Wyeth Ayerst) |
| | CCI-779 (mTOR-Kinase-Inhibitor, Wyeth) | |
| | | PG2 (Hämatopoese-Verstärker, Pharmagenesis) |
| | Exisulind (PDE-V-Inhibitor, Cell Pathways) | |
| | | Immunol™ (Triclosan-Oralspülung, Endo) |
| | CP-461 (PDE-V-Inhibitor, Cell Pathways) | |
| | | Triacetyluridin (Uridin-Prodrug, Wellstat) |
| | AG-2037 (GART-Inhibitor, Pfizer) | |
| | WX-UK1 (Plasminogenaktivator-Inhibitor, Wilex) | SN-4071 (Sarkom-Mittel, Signature BioScience) |
| | | |
| | PBI-1402 (PMN-Stimulans, ProMetic LifeSciences) | TransMID-107™ (Immunotoxin, KS Biomedix) |
| | | |
| | Bortezomib (Proteasom-Inhibitor, Millennium) | PCK-3145 (Apoptose-Förderer, Procyon) |
| | SRL-172 (T-Zell-Stimulans, SR Pharn | Doranidazol (Apoptose-Förderer, Pola) |
| | TLK-286 (Glutathion-S-Transferase-Inhibitor, Telik) | |
| | | CHS-828 (cytotoxisches Mittel, Leo) |
| | PT-100 (Wachstumsfaktor-Agonist, Point Therapeutics) | |
| | | trans-Retinsäure (Differentiator, NIH) |
| | Midostaurin (PKC-Inhibitor, Novartis) | |
| | | MX6 (Apoptose-Förderer, MAXIA) |
| | Bryostatin-1 (PKC-Stimulans, GPC Biotech) | |
| | | Apomin (Apoptose-Förderer, ILEX Oncology) |
| | CDA-II (Apoptose-Förderer, Everlife) | |
| | | Urocidin (Apoptose-Förderer, Bioniche) |
| | SDX-101 (Apoptose-Förderer, Salmedix) | |
| | | Ro-31-7453 (Apoptose-Förderer, La Roche) |
| | Ceflatonin (Apoptose-Förderer, ChemGenex) | |
| | | Brostallicin (Apoptose-Förderer, Pharmacia) |
| | | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

Gegenstand der Erfindung sind Verbindungen der Formel I gemäß Anspruch 1 sowie ihrer pharmazeutisch verwendbaren Salze, Tautomeren und Stereoisomeren, einschließlich deren Mischungen in allen Verhältnissen, zur Verwendung zur Behandlung von Tumoren, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, Autoimmunerkrankungen, Zirrhose, Diabetes und Erkrankungen der Blutgefässe.

### ASSAYS

Die in den Beispielen beschriebenen erfindungsgemäßen Verbindungen wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

### FAK- Kinase Assay (autophosphorylation)

Der Fokale-Adhäsionskinase(FAK)-Assay wird entweder als 384-Well-Flashplate-Assay (z. B. für Topcount-Messungen) oder als 384-Well-Image-Flashplate-Assay (für LEADseeker-Messungen) durchgeführt. 2 nM FAK, 400 nM biotinyliertes Substrat (His-TEV-hsFAK (31 686)(K454R) x Biotin) und 1 µM ATP (versetzt mit 0,25 Ci 33P-ATP/Well) werden in einem Gesamtvolumen von 50 µl (60 mM Hepes, 10 mM MgCl₂, 1,2 mM Dithiothreitol, 0,02 % Brij35, 0,1 % BSA , pH 7.5) mit oder ohne Testverbindung 2 Stunden lang bei 30 °C inkubiert. Die Reaktion wird mit 25 µl 200 mM EDTA gestoppt. Nach 30 Min bei 30 °C wird die Flüssigkeit entfernt und jeder Well dreimal mit 100 µl 0,9% Natriumchloridlösung gewaschen. Nicht-spezifische Reaktion wird in Gegenwart von 1 µM EMD 1076893/0(PF--562271) bestimmt. Die Radioaktivität wird mit Topcount (bei Verwendung von Flashplates) bzw. mit LEADseeker (bei Verwendung von Image-Flashplates) gemessen. Ergebnisse (z. B. IC50-Werte) werden mit z. B. einem Symyx Assay Explorer berechnet.

### Methode zur zellulären Testung von FAK-Kinase-Inhibitoren

Zur Analyse der zellulären Aktivität von FAK wird das Maß der Autophosphorylierung von FAK an Tyrosin 397 mit Hilfe eines Luminex-basierten Assays im 96-well Format bestimmt. HT29 Zellen werden mit 30.000 Zellen pro well in 100 µl Medium (90% DMEM / 10% FCS) ausgesät und am folgenden Tag für 30 min mit einer seriellen Verdünnung der Prüfsubstanz (7 Konzentrationen) unter serumfreien Bedingungen inkubiert. Im Anschluss werden die Zellen mit 90 µl Lysepuffer (20mM Tris/HCl pH 8,0, 150mM NaCl, 1% NP40, 10% Glycerol, 1% Phosphatase-Inhibitor II, 20mM β-Glycerolphosphat, 0,1% Protease-Inhibitor Cocktail III, 0,01 % Benzonase) pro well lysiert, und die Lysate werden mittels Zentrifugation durch eine 96-well Filterplatte (0,65 µm) von unlöslichen Zellbestandteilen abgetrennt. Die Lysate werden üN bei 4°C mit Luminex-Beads, an die ein anti-total FAK Antikörper gekoppelt ist, unter Schütteln inkubiert. Am folgenden Tag erfolgt die Detektion durch Zugabe eines P-Y397-FAK Antikörpers sowie eines speziesspezifischen PE-markierten Sekundärantikörpers. Der Nachweis von P-Y397-FAK erfolgt durch Messung im Luminex100 Gerät durch Bestimmung von 100 Ereignissen pro Kavität in 60 sec Messzeit. Als pharmakologischer Blank werden die erhaltenen Signale von Zellen, die mit 30 µM eines FAK Referenz-Inhibitors behandelt wurden, von allen anderen Ansätzen abgezogen. Als Kontrollwert der maximalen Phosphorylierung von FAK an Y397 werden die Signale von Zellen, die nur mit dem Lösungsmittel (0,3% DMSO) behandelt wurden, verwendet. Die Werte der mit Prüfsubstanz behandelten Ansätze werden hiervon als Prozent von Kontrolle berechnet und IC50 Werte werden mittels Assay Explorer ermittelt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | EI (Elektronenstoß-Ionisation) M⁺ |
| | FAB (Fast Atom Bombardment) (M+H)⁺ |
| | ESI (Electrospray lonization) (M+H)⁺ (wenn |
| | nichts anderes angegeben) |

%

### HPLC-MS Bedingungen:

1. Säule : Acquity BEH C-18 (2.1 x 100 mm , 1.7 µm)
2. Mobile Phase : A - 5 mM Ammoniumacetate in Wasser B Acetonitril
3. Flow Mode : Gradient
Time
Point
Flow Rate
(mL/min)

| % | A | % | B |
|---|---|---|---|
| 0.0 | 0.3 | 90 | 10 |
| 1.0 | 0.3 | 90 | 10 |
| 2.0 | 0.3 | 85 | 15 |
| 4.5 | 0.3 | 45 | 55 |
| 6.0 | 0.3 | 10 | 90 |
| 8.0 | 0.3 | 10 | 90 |
| 9.0 | 0.3 | 90 | 10 |
| 10.0 | 0.3 | 90 | 10 |

4. Flow : 0.3 ml/min.
5. UV max. : 254.0 nm
6. Säulentemperatur : 30.0 deg.
7. Sample Preparation: Acetonitril + Wasser

*HPLC: La Chrom Anlage
Chromolite Performance RP18-e 100-4,6mm
Gradient: ACN/H2O mit 0,01% Ameisensäure
Methode: Chromolith/Chromolith (extended)
Fluß: 3mL/min

### $

Säule: XBridge C8, 3.5 µm, 4.6 x 50 mm;
Lösungsmittel A: Wasserr + 0.1 % TFA;
Lösungsmittel B: Acetonitril + 0.1 % TFA;
Flow: 2 ml/min;
Gradient: 0 min: 5 % B, 8 min: 100 % B, 8.1 min: 10 %; 254 nm

### §

NMR Spektrum nach Zugabe von deuterierter Trifluoressigsäure

### Beispiele

### Herstellung von N-(2-{2-[2-(4-Methansulfonyl-phenylamino)-pyrimidin-4-yl]-ethyl}-phenyl)-N-methyl-methansulfonamid ("A1")

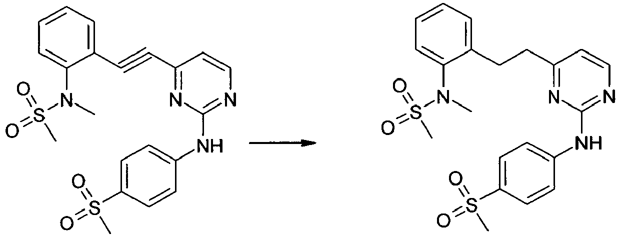

100 mg N-(2-{2-[2-(4-Methansulfonyl-phenylamino)-pyrimidin-4-yl]-ethinyl}-phenyl)-N-methyl-methansulfonamid werden in 1 ml Methanol gelöst und mit 100 mg Aktivkohle (mit 10% Pd) versetzt. Man erzeugt eine Wasserstoffatmosphäre und rührt die Suspension für 3 h bei Raumtemperatur. Nachfolgend wird der Katalysator abfiltriert und das Lösungsmittel entfernt. Der Rückstand wird über Kieselgel chromatographiert. Man erhält 50 mg der Titelverbindung als farblosen Feststoff;
¹H NMR (500 MHz, DMSO-d₆) δ [ppm] J [Hz]:
10.14 (s, 1H), 8.44 (d, J = 5.0, 1H), 8.02 (d, J = 8.9, 2H), 7.79 (d, J = 8.9, 2H), 7.52 - 7.46 (m, 1H), 7.42 - 7.36 (m, 1H), 7.36 - 7.26 (m, 2H), 6.86 (d, J = 5.0, 1H), 3.13 (s, 3H), 3.07 (s, 3H), 2.98 (m, 4H).

### Herstellung von N-Methyl-N-(2-{2-[2-(2-oxo-1,2,3,4-tetrahydro-chinolin-6-ylamino)-pyrimidin-4-yl]-ethyl}-phenyl)-methansulfonamid ("A2")

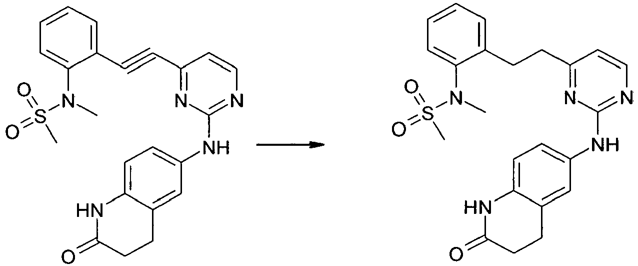

Man löst 200 mg N-{2-[2-(2-Chlor-pyrimidin-4-yl)-ethyl]-phenyl}-N-methyl-methanesulfonamid und 70 mg 6-Amino-3,4-dihydrochinolin-2(1H)-on in 3 ml n-Butanol und erwärmt in einem geschlossenen Gefäß für 12 h auf 120°C. Nach dem Abkühlen auf Raumtemperatur wird das Lösungsmittel im Vakuum entfernt und der Rückstand über Kieselgel chromatographiert. Man erhält 50 mg der Titelverbindung als farblosen Feststoff;
¹H NMR (500 MHz, DMSO-d₆) d [ppm] J [Hz]:
9.93 (s, 1H), 9.39 (s, 1H), 8.29 (d, J = 5.0, 1H), 7.57 (s, 1H), 7.52 - 7.45 (m, 2H), 7.40 - 7.36 (m, 1H), 7.34 - 7.27 (m, 2H), 6.76 (d, J = 8.6, 1H), 6.65 (d, J = 5.0, 1H), 3.12 (s, 3H), 3.07 (s, 3H), 2.91 (m, 2H), 2.83 (t, J = 7.5, 2H), 2.45 - 2.38 (m, 2H).

### Herstellung von 3-(3-{2-[2-(2-Oxo-2,3-dihydro-1H-indol-5-ylamino)-pyrimidin-4-yl]-ethyl}-pyridin-2-yl)-azetidin-3-carbonitril ("A16")

1) 3-Cyan-3-(3-iod-pyridin-2-yl)-azetidin-1-carbonsäure-tert.-butylester 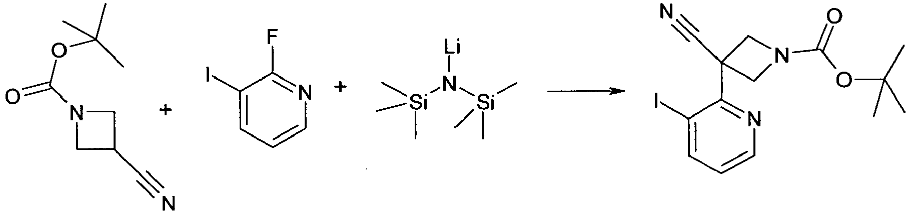
   Unter Stickstoff wird das Cyanazetidin (70 mg, 0.4 mmol) und 2-Fluor-3-iodpyridin (93 mg, 0.4 mmol) in trockenem THF (18 ml) gelöst. LiHMDS (Lithium-hexamethyldisilazid) in THF (0.7 ml, 0.7 mmol) wird bei RT zu der Lösung gegeben und für weitere 45 min gerührt. Dann gibt man den Ansatz auf gesättigte wässrige Ammoniumchlorid Lösung und extrahiert erschöpfend mit Essigester. Nach dem Trocknen der organischen Phase über Natriumsulfat und der Chromatographie des organischen Rückstandes an Kieselgel (Petrolether/Essigester = 8:2) erhält man das Produkt mit 76% Ausbeute (110 mg);
   ¹H NMR (DMSO-d₆) δ [ppm] J [Hz]:
   ppm 8.65 (dd, 1 H) 8.44 (dd, 1 H) 7.27 (dd, 1 H) 4.63 (d, 2 H) 4.49 (d, 2 H) 1.39 (s, 9 H).
2) 3-Cyan-3-(3-trimethylsilanylethinyl-pyridin-2-yl)-azetidin-1-carbonsäure-tert.-butylester 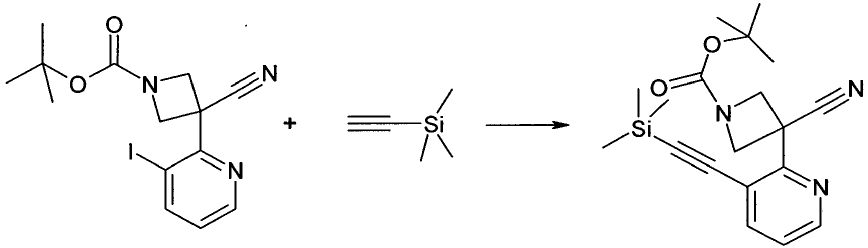
   Unter Stickstoff wird das Iod-pyridin (790 mg, 2 mmol) in 10 ml Acetonitril gelöst, 10 ml Triethlyamin und 40 mg Kupfer(I)iodid werden zugefügt. Diese Mischung wird für 10 Minuten entgast. Nachfolgend fügt man 72 mg des Katalysators (Pd(PPh₃)₂Cl₂) und das Alkin (260 mg, 2.6 mmol) hinzu. Der Ansatz wird für 2 h bei 80°C gerührt. Das Lösungsmittel wird entfernt, der Rückstand mit Essigester aufgenommen und der entstehende Niederschlag durch Filtration abgetrennt. Die organische Phase wird mit Wasser und gesättigter NaCl-Lösung gewaschen, getrocknet und mit Petrolether / Essigester = 8:2 über Kieselgel chromatographiert. Man erhält 670 mg des Produkts (91%);
   ¹H NMR (DMSO-d₆) δ [ppm] J [Hz]:
   8.63 (dd, 1 H), 8.04 (dd, 1 H), 7.52 (dd, 1 H), 4.64 (d, 2 H), 4.44 (d, 2 H), 1.39 (s, 9 H), 0.29 (s, 9 H).
3) 3-Cyan-3-(3-ethinyl-pyridin-2-yl)-azetidin-1-carbonsäure-tert.-butylester 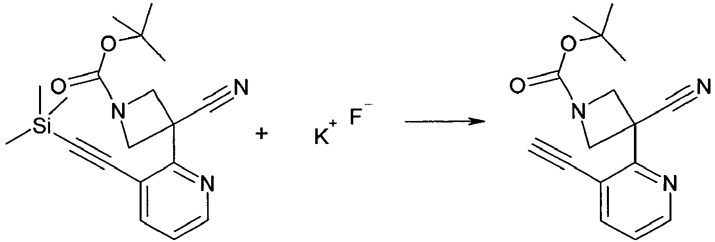 Man löst 670 mg (1.9 mmol) der Silyl-Verbindung in 15 ml Methanol und fügt 220 mg (3.8 mmol) Kaliumfluorid hinzu. Nach 2 h bei RT ist die Reaktion beendet. Man entfernt das Lösungsmittel im Vakuum und chromatographiert das Rohprodukt über Kieselgel (Petrolether/Essigester = 8:2) und erhält 500 mg (94%) des gewünschten Produkts;
   ¹H NMR (DMSO-d₆) δ [ppm] J [Hz]:
   8.65 (dd, 1 H), 8.09 (dd, 1 H), 7.54 (dd, 1 H), 4.95 (s, 1 H), 4.65 (d, 2H), 4.46 (d, 2 H), 1.39 (s, 9 H).
4) 3-[3-(2-Chlor-pyrimidin-4-ylethinyl)-pyrimidin-2-yl]-3-cyan-azetidin-1-carbonsäure-tert.-butylester 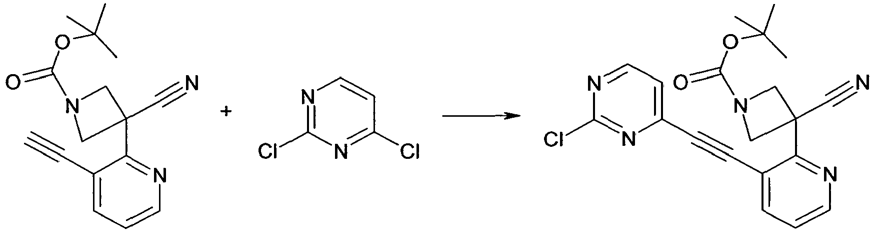
   Man löst 316 mg (2.1 mmol) 2,4-Dichlorpyrimidin in 5 ml Acetonitril und 5 ml Triethylamin. Nach dem Zusatz von 34 mg Kupfer(I)iodid wird für 10 min entgast. Man gibt 62 mg Pd(PPh₃)₂Cl₂ und 500 mg des zuvor hergestellten Alkins hinzu und erwärmt auf 80°C für 2 h. Nach dem Entfernen des Lösungsmittels im Vakuum wird der Rückstand in Essigester aufgenommen, von Feststoffen befreit, mit Wasser und gesättigter NaCl-Lösung gewaschen, getrocknet und nach erneutem Eindampfen an Kieselgel chromatogrphiert (Petrolether/Essigester = 6:4). Man erhält 470 mg des gewünschten Produkts (67%);
   ¹H NMR (DMSO-d₆) δ [ppm] J [Hz]:
   8.93 (d, 1 H), 8.77 (dd, 1 H), 8.31 (dd, 1 H), 7.86 (d, 1 H), 7.66 (dd, 1 H), 4.73 (d, 2 H), 4.59 (d, 2 H), 1.39 (s, 9 H)
5) 3-[3-(2-Chlor-pyrimidin-4-ylethyl)-pyrimidin-2-yl]-3-cyan-azetidin-1-carbonsäure-tert.-butylester 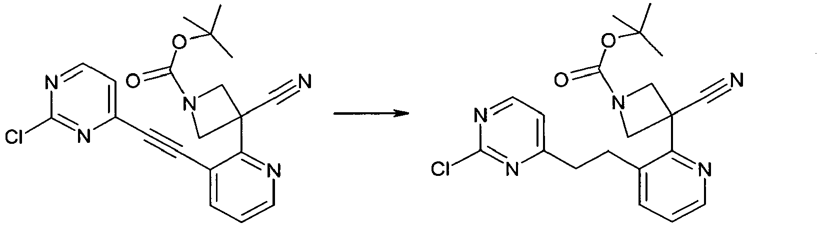
   Man löst 250 mg (0.6 mmol) des Ausgangsmaterials in 25 ml Essigester und hydriert bei 10 bar mit einem Fluß von 0.5 ml/min am H-cube (Hydrierreaktor der Firma Thales Nanotechnology) bis zur vollständigen Umsetzung. Nach Entfernen des Lösungsmittels und Chromatographie an Kieselgel mit Petrolether/Essigester = 6:4 erhält man 80 mg (31%) des gewünschten Produkts;
   ¹H NMR (DMSO-d₆) δ [ppm] J [Hz]:
   8.68 (d, 1 H) 8.49 (dd, 1 H) 7.91 (dd, 1 H) 7.37 - 7.63 (m, 2 H) 4.66 (d, 2 H) 4.52 (d, 2 H) 3.21 (m, 2 H) 2.97 (m, 2 H) 1.39 (s, 9 H).
6) 3-Cyan-3-(3-{2-[2-(2-oxo-2,3-dihydro-1H-indol-5-ylamino)-pyrimidin-4-yl]-ethyl}-pyridin-2-yl)azetidin-1-carbonsäure-tert.-butylester 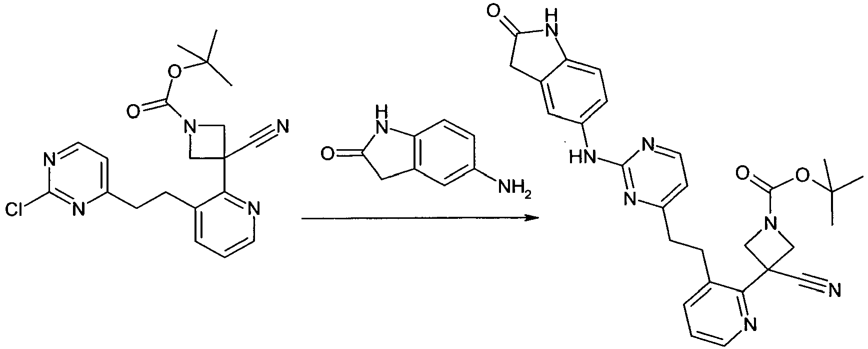
   Man löst 80 mg (0.2 mmol) des Pyrimidins in 1 ml n-Butanol und fügt das Oxindol (30 mg, 0.2 mmol) hinzu. Dann erhitzt man die Mischung für 6 h auf 120°C. Nach dem Entfernen des Lösungsmittels wird der Ansatz mit Dichlormethan/Methanol = 98:2 an Kieselgel chromatographisch aufgereinigt. Man erhält das Produkt in 18% Ausbeute und setzt es ohne weitere Charakterisierung im nächsten Schritt ein.
7) 3-(3-{2-[2-(2-Oxo-2,3-dihydro-1H-indol-5-ylamino)-pyrimidin-4-yl]-ethyl}-pyridin-2-yl)-azetidin-3-carbonitril ("A16") 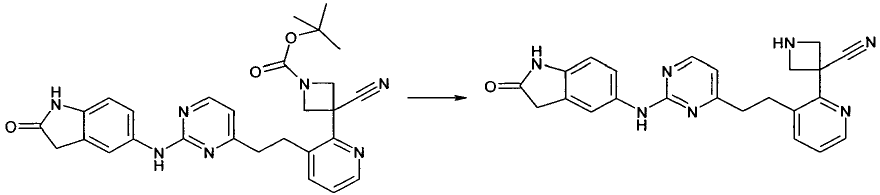
   Man löst 18 mg der Ausgangsverbindung in 0.5 ml Dichlormethan und gibt 60 mg Trifluoressigsäure hinzu. Nach 8 h bei RT ist die Reaktion vollständig. Man reinigt den Ansatz an Kieselgel mit Dichlormethan/Methanol/NH₄OH = 95/4.5/0.5 auf und erhält 8 mg "A16";
   ¹H NMR (500 MHz, DMSO-d₆) δ [ppm] J [Hz]:
   10.20 (s, 1 H) 9.73 (br. s., 1 H) 9.33 (s, 1 H) 9.06 (br. s., 1 H) 8.54 (dd, 1 H) 8.34 (d, 1 H) 7.96 (dd, 1 H) 7.65 (d, 1 H) 7.42 - 7.61 (m, 2 H) 6.75 (d, 1 H) 6.74 (d, 1 H) 4.83 (d, 2 H) 4.74 (d, 2 H) 3.46 (s, 2 H) 2.89 - 3.10 (m, 4 H) 3,93 min.

### Herstellung von (5-Fluor-2-{2-[2-(2-oxo-2,3-dihydro-1H-indol-5-ylamino)-pyrimidin-4-yl]-ethyl}-phenyl)-acetonitril ("A18")

1) 2-(2-((Triethylsilyl)ethinyl)-4-(trifluormethyl)phenyl)acetonitril 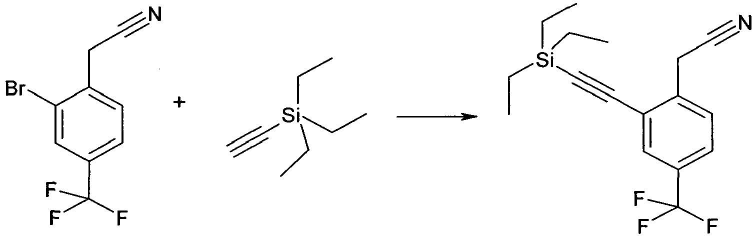
   Bis(triphenylphosphin)palladium(II)chlorid (0.106 g, 0.151 mmol), Kupfer(I)iodid (0.058 g, 0.303 mmol) und Triphenylphospin (0.079 g, 0.303 mmol) werden in einer Mischung aus i-Pr₂NH (11.36 ml)/DMF (3.79 ml) (mit N₂ für 15 min entgast) gelöst. Zur resultierenden orangefarbenen Lösung wird 2-(2-Brom-4-(trifluormethyl)phenyl)acetonitril (1.1 g, 3.8 mmol) hinzu gegeben. Die Mischung wird für eine Stunde bei RT gerührt. Triethyl(ethinyl)silan (1.018 ml, 5.68 mmol) wird tropfenweise zugesetzt und der Ansatz auf 80°C erwärmt. Eine gesättigte, wässrige NH₄Cl Lösung (50 ml) wird nach 1 Stunde zu dem stark dunkel gefärbten Ansatz gegeben. Nach der Phasentrennung wird die wässrige Phase dreimal mit je 50 ml Essigester extrahiert. Die vereinigten organischen Phasen werden über Na₂SO₄ getrocknet und im Vakuum vollständig eingedampft. Der Rückstand wird über Kieselgel chromatographisch mit einer 98:2 Mischung aus Petrolether und Essigester aufgereinigt.
   Man erhält 2-(2-((Triethylsilyl)ethinyl)-4-(trifluoromethyl)phenyl)acetonitril (orangefarbenes Öl, 91%, 1.226 g, 3.79 mmol);
   ¹H NMR (DMSO-d₆) δ [ppm] J [Hz]: 7.80 - 7.89 (m, 2 H) 7.73 - 7.79 (m, 1 H) 4.17 (s, 2 H) 1.04 (t, 9 H) 0.62- 0.81 (m, 6 H).
2) 2-(2-Ethinyl-5-fluorophenyl)acetonitril 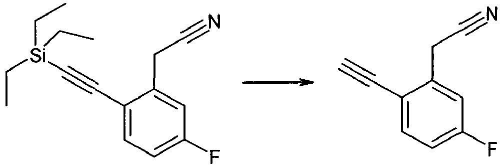
   2-(5-Fluor-2-((triethylsilyl)ethinyl)phenyl)acetonitril (1.044 g, 3.82 mmol) wird in Methanol (38.2 ml) gelöst und Kaliumfluorid (0.887 g, 15.27 mmol) zugesetzt. Die gelbliche Lösung wird für 48 Stunden bei RT gerührt. Nachfolgend entfernt man das Lösungsmittel im Vakuum und nimmt den Rückstand mit Wasser (30 ml) auf. Nach dem unter 1) beschriebenen üblichen Extraktions-und Trocknungsverfahren erhält man in quantitativer Ausbeute 606 mg der Titelverbindung. (3.81 mmol);
   ¹H NMR (DMSO-d₆) δ [ppm] J [Hz]:
   7.63 (dd, 1 H) 7.38 (dd, 1 H) 7.26 (td, 1 H) 4.58 (s, 1 H) 4.09 (s, 2 H).
3) 2-(2-((2-Chlorpyrimidin-4-yl)ethynyl)-5-fluorphenyl)acetonitril 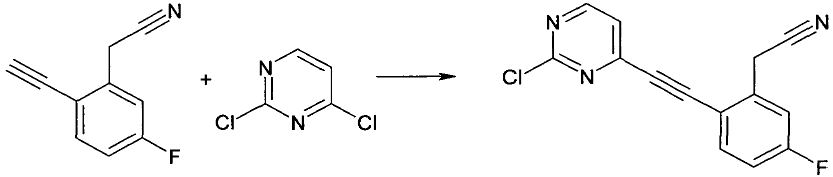
   2,4-Dichlorpyrimidin (764 mg, 5.13 mmol) wird in entgastem Acetonitril (19.700 ml) und Triethylamin (19.70 ml) gelöst. Man gibt Kupfer(I)iodid (75 mg, 0.395 mmol) hinzu, entgast erneut für 10 min. und addiert dann nacheinander zu der grünlichen Lösung 2-(2-Ethinyl-5-fluorophenyl)acetonitril (628 mg, 3.95 mmol) sowie Bis(triphenylphosphin)palladium(II)chlorid (138 mg, 0.197 mmol). Die Reaktionsphase wird für 30 min. auf 80°C erwärmt. Nachfolgend wird, wie oben beschrieben, aufgearbeitet und die erhaltene Rohmasse an Kieselgel chromatographisch aufgereinigt (Petrolether/Essigester = 80/20 bis 75/25). Man erhält als orangefarbenen Feststoff 700 mg 2-(2-((2-Chloropyrimidin-4-yl)ethinyl)-5-fluorophenyl)-acetonitril (2.58 mmol, 65.3 % Ausbeute);
   ¹H NMR (DMSO-d₆) δ [ppm] J [Hz]:
   8.89 (d, 1 H) 7.87 (d, 1 H) 7.83 - 7.90 (m, 1 H) 7.48 (dd, 1 H) 7.38 (td, 1H) 4.29 (s, 2 H).
4) 2-(2-(2-(2-Chlorpyrimidin-4-yl)ethyl)-5-fluorophenyl)acetonitril 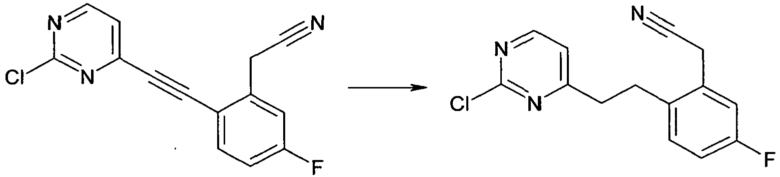
   2-(2-((2-Chlorpyrimidin-4-yl)ethinyl)-5-fluorphenyl)acetonitril (0.695 g, 2.56 mmol) wird in Essigester (90 ml) gelöst und Pd/C 10% (0.142 g, 0.133 mmol) werden zugesetzt. Die Suspension wird bei RT unter 40 psi H₂-Druck in einem Parr Apparat (Hydriervorrichtung) für 24 h hydriert. Da der Umsatz sehr schleppend verläuft, werden 9 ml Methanol und weiterer Katalysator zugesetzt (Pd/C 10% (0.142 g, 0.133 mmol). In weiteren 28 h werden drei weitere Male je 142 mg Katalysator zugefügt, bis ein vollständiger Umsatz zu beobachten ist.
   Die dunkle Suspension wird filtriert, mit Essigester und Methanol gewaschen und das Filtrat zur Trockene im Vakuum eingedampft. Nach Chromatographie der 700 mg Rohprodukt an Kieselgel mit Petrolether/Essigester = 7:3 erhält man 386 mg 2-(2-(2-(2-Chlorpyrimidin-4-yl)ethyl)-5-fluorphenyl)acetonitril (1.400 mmol, 54.7 % yield) als gelbliches Öl;
   ¹H NMR (DMSO-d₆) δ [ppm] J [Hz]:
   8.66 (d, 1 H) 7.49 (d, 1 H) 7.31 (dd, 1 H) 7.22 (dd, 1 H) 7.13 (td, 1 H) 4.13 (s, 2 H) 2.91 - 3.18 (m, 4 H).
5) "A18" 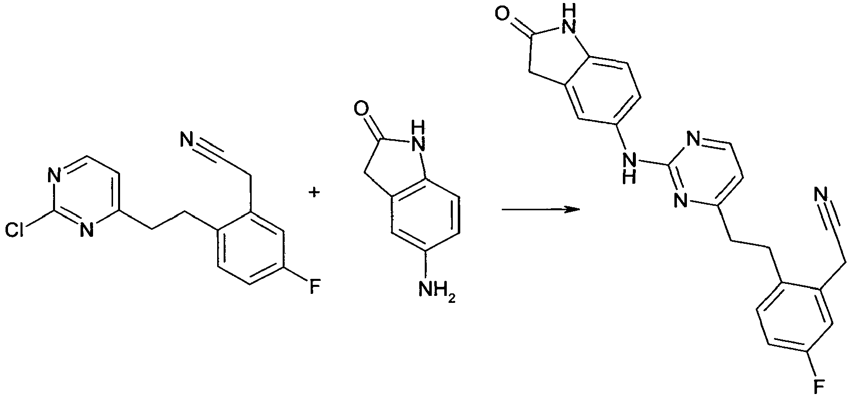
   2-(2-(2-(2-Chlorpyrimidin-4-yl)ethyl)-5-fluorphenyl)acetonitril (200 mg, 0.725 mmol) wird in Buthanol (6.3 ml) gelöst und 5-Aminoindolin-2-on (107 mg, 0.725 mmol) hinzugefügt. Die braune Suspension wird für 6.5 h in einem geschlossenen Gefäß auf 120°C erwärmt.
   Nach dem Abkühlen auf RT wird Diethylether (15 ml) zugesetzt und die Suspension über einen Büchner-Trichter filtriert und der zurückbleibende Feststoff zunächst mit Ether gewaschen und dann an Kieselgel mit Dichlormethan/Methanol = 95:5 bis 93.7 aufgereinigt. Man erhält 142 mg "A18" als gelblich-braunen Feststoff. (0.367 mmol, 50.5 % Ausbeute);
   ¹H NMR (500 MHz, DMSO-d₆) δ [ppm] J [Hz]:
   10.19 (s, 1 H) 9.35 (s, 1 H) 8.30 (d, 1 H) 7.66(d, 1 H) 7.52 (dd, 1 H) 7.31 (dd, 1 H) 7.22 (dd, 1 H) 7.13 (td, 1 H) 6.73 (d, 1 H) 6.69 (d, 1 H) 4.10 (s, 2 H) 3.46 (s, 2 H) 2.97 - 3.09 (m, 2 H) 2.83 - 2.96 (m, 2 H).

### Herstellung von 1-(2-{2-[2-(2-Oxo-2,3-dihydro-1H-indol-5-ylamino)-pyrimidin-4-yl]-ethyl}-4-trifluormethyl-phenyl)-cyclobutancarbonitril ("A19")

1) (4-Trifluormethyl-phenyl)-cyclobutancarbonitril 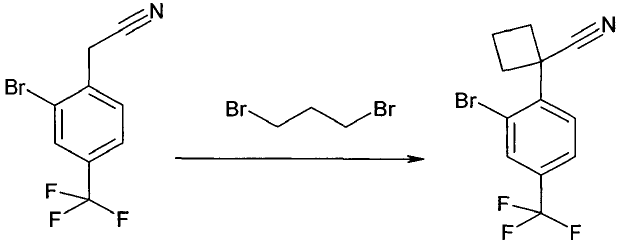
   Natriumhydrid (380 mg; 9.5 mmol; Gehalt 60%) wird in 4 ml DMSO suspendiert. 1,3-Dibromopropan (920 mg; 4.5 mmol) und (2-Brom-4-trifluormethyl-phenyl)-carbonitril werden in 10 ml DMSO gelöst und tropfenweise zu der Natriumhydrid-Suspension gegeben. Der Ansatz wird für 12 h bei RT gerührt. Dann werden 10 ml Wasser zugegeben und die Mischung mit Essigester erschöpfend extrahiert. Nach dem üblichen weiteren Aufarbeiten und Aufreinigen an Kieselgel (Petrolether/Essigester = 95:5) erhält man (4-Trifluormethyl-phenyl)-cyclobutancarbonitril (965 mg, 84%);
   ¹H NMR (DMSO-d₆) δ [ppm] J [Hz]:
   8.09 (d, 1 H), 7.85 (dd, 1 H), 7.68 (d, 1 H), 2.81 - 3.03 (m, 2 H), 2.65 - 2.81 (m, 2 H), 2.18 - 2.44 (m, 1 H), 1.82 - 2.00 (m, 1 H).
2) 1-(2-Triethylsilanylethinyl-4-trifluormethyl-phenyl)-cyclobutancarbonitril 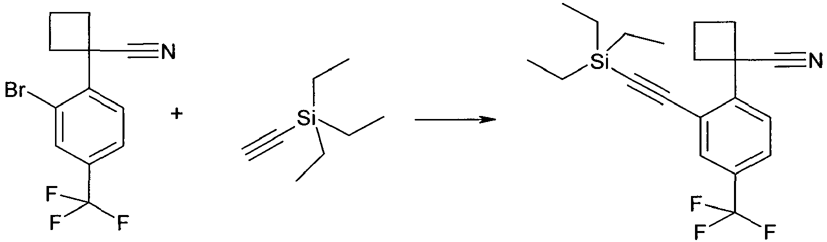
   Pd(PPh₃)₂Cl₂ (71 mg), Kupfer(I)iodid (38 mg) und PPh₃ (52 mg) werden in entgastem iPr₂NH/DMF = 3:1 suspendiert. Das Arylbromid (750 mg, 2.5 mmol) wird zugesetzt und nach einer Stunde fügt man zu der roten Lösung 0.7 ml (3.7 mmol) Ethinylsilan und erhitzt für 18 h auf 80°C. Der Ansatz wird mit 150 ml Essigester verdünnt, zweimal mit 100 ml NH₄Cl-Lösung und einmal mit 100 ml gesättigter NaCl Lösung gewaschen. Die organische Phase wird getrocknet, eingedampft und der Rückstand an Kieselgel chromatographisch aufgereinigt (Petrolether/Essigester =9:1). Man erhält 506 mg der Titelverbindung (56%);
   ¹H NMR (DMSO-d₆) δ [ppm] J [Hz]:
   7.79 - 7.87 (m, 2 H) 7.66 (d, 1 H) 2.63 - 3.00 (m, 4 H) 2.18 - 2.43 (m, 1 H) 1.80 - 2.07 (m, 1 H) 0.94 - 1.13 (m, 9 H) 0.62 - 0.80 (m, 6 H).
3) 1-(Ethinyl-4-trifluoromethyl-phenyl)-cyclobutancarbonitril 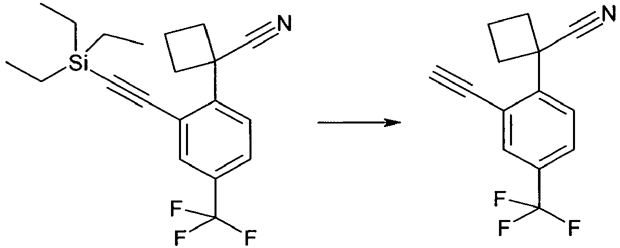
   Unter Stickstoff wird das Ausgangsmaterial (500 mg, 1.4 mmol) in 5 ml Methanol gelöst und mit 320 mg (5.7 mmol) Kaliumfluorid versetzt. Nach 16 h bei RT wird das Lösungsmittel entfernt und der Rückstand in Essigester (200 ml) gelöst. Nach dem Waschen mit gesättigter NaCl-Lösung (2*50 ml) wird die organische Phase getrocknet und der Rückstand mit Dichlormethan an Kieselgel chromatographiert. Man erhält 311 mg (90%) der gewünschten Verbindung;
   ¹H NMR (DMSO-d₆) δ [ppm] J [Hz]:
   7.90 (d, 1 H) 7.84 (dd, 1 H) 7.66 (d, 1 H) 4.80 (s, 1 H) 2.81 - 2.94 (m, 2H) 2.66 - 2.81 (m, 2 H) 2.21 - 2.42 (m, 1 H) 1.85 - 2.02 (m, 1 H).
4) 1-[2-(2-Chlor-pyrimidin-4-ylethinyl)-5-fluorphenyl)-4-trifluormethyl-phenyl]-cyclobutancarbonitril 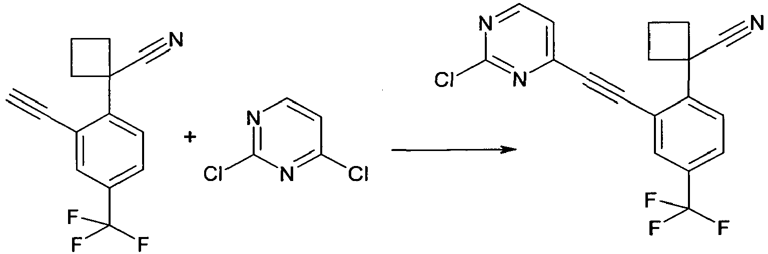
   2,4-Dichlorpyrimidin (251 mg, 1.7 mmol) wird in entgastem Acetonitril (5 ml) und Triethylamin (5 ml) gelöst. Man gibt Kupfer(I)iodid(24 mg) hinzu, entgast erneut für 10 min. und addiert dann nacheinander zu der grünlichen Lösung das Ethin (311 mg, 1.2 mmol) sowie Bis(triphenylphosphin)palladium(II)chlorid (44 mg). Die Reaktionsphase wird für 30 min. auf 80°C erwärmt. Nachfolgend wird, wie oben beschrieben, aufgearbeitet und die erhaltene Rohmasse an Kieselgel chromatographisch aufgereinigt (Petrolether / Essigester = 80/20). Man erhält das Produkt als orangefarbenen Feststoff (256 mg, 0.7 mmol, 57 % Ausbeute).
   ¹H NMR (DMSO-d₆) δ [ppm] J [Hz]:
   8.92 (d, 1 H) 8.19 (d, 1 H) 7.94 - 8.03 (m, 1 H) 7.84 (d, 1 H) 7.77 (d, 1H) 2.90 - 3.09 (m, 2 H) 2.75 - 2.90 (m, 2 H) 2.31 - 2.45 (m, 1 H) 1.84 - 2.08 (m, 1 H).
5) 1-[2-(2-Chlor-pyrimidin-4-ylethyl)-5-fluorphenyl)-4-trifluormethyl-phenyl]-cyclobutancarbonitril 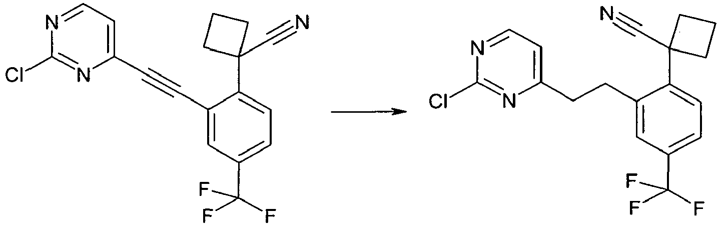
   Das Ausgangsmaterial (256 mg, 0.7 mmol) wird in Essigester (20 ml) gelöst und am H-Cube bei 20 bar und 30°C (Flußrate 0.8 ml/min) über eine Pd/C (10%) Kartusche in drei Läufen hydriert.
   Die dunkle Suspension wird filtriert, mit Essigester und Methanol gewaschen und das Filtrat zur Trockene im Vakuum eingedampft. Nach Chromatographie an Kieselgel mit Petrolether/Essigester = 7:3 erhält man 202 mg der Titelverbindung (0.5 mmol, 78 % yield) als gelblichen Feststoff;
   ¹H NMR (DMSO-d₆) δ [ppm] J [Hz]:
   8.69 (d, 1 H) 7.73 (d, 1 H) 7.65 (dd, 1 H) 7.54 (d, 1 H) 7.50 (d, 1 H)3.15 - 3.23 (m, 2 H) 3.03 - 3.15 (m, 2 H) 2.79 - 2.93 (m, 2 H) 2.68 - 2.79 (m, 2 H) 2.29 - 2.45 (m, 1 H) 1.83 -2.05 (m, 1 H).
6) "A19" 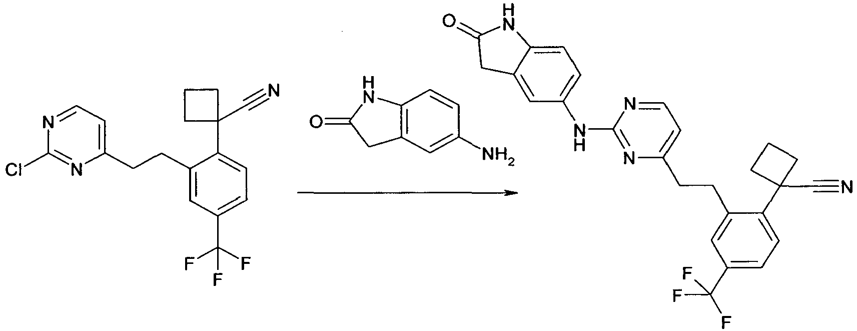
   In einem Mikrowellengefäß werden die Ausgangsmaterialien in 5 ml n-Butanol gelöst und für 6 h auf 120°C erwärmt. Nachfolgend rührt der Ansatz noch für 12 h bei RT. Da noch Ausgangsmaterial zu detektieren ist, wird erneut für 3h auf 120°C erhitzt. Anschließend entfernt man das Lösungsmittel im Vakuum und reinigt den Rückstand an Kieselgel auf (Dichlormethan/Methanol = 99:1). Der Rückstand wird mit Diethylether gefällt und man erhält 65 mg "A19";
   ¹H NMR (500 MHz, DMSO-d₆) δ [ppm] J [Hz]:
   10.20 (s, 1 H) 9.33 (s, 1 H) 8.33 (d, 1 H) 7.47 - 7.79 (m, 5 H) 6.76 (d, 1 H) 6.76 (d, 1 H) 3.45 (s, 2 H) 2.93 - 3.19 (m, 4 H) 2.65 - 2.93 (m, 4 H) 2.28 - 2.45 (m, 1 H) 1.79 - 2.07 (m, 1 H).

**Analog werden die nachstehenden Verbindungen erhalten**

| Nr. | Name / Struktur | ¹H NMR (500 MHz, DMSO-d₆) δ [ppm] J [Hz] |
|---|---|---|
| | | |
| "A3" | 6-{4-[2-(3-Methansulfonyl-phenyl)-ethyl]-pyrimidin-2-ylamino}-3,4-dihydro-1H-chinolin-2-on | 9.94 (s, 1H), 9.40 (s, 1H), 8.30 (d, J = 5.0, 1H), 7.80 (s, 1H), 7.75 (d, J = 7.5, 1H), 7.63 - 7.54 (m, 3H), 7.49 (dd, J = 8.5, 2.3, 1H),6.75(d,J = 8.6, 1H), 6.70 (d, J = 5.0, 1H), 3.17 (s, 3H), 3.13 (m, 2H), 2.96 (m, 2H), 2.83 (m, 2H), 2.45 - 2.38 (m, 2H) |
| | 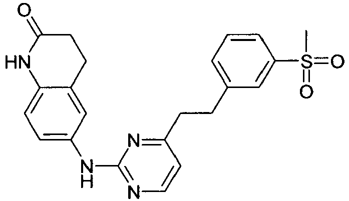 | |
| "A4" | (4-Methansulfonyl-phenyl)-{4-[2-(3-methansulfonyl-phenyl)-ethyl]-pyrimidin-2-yl}-amin | 10.16 (s, 1H), 8.45 (d, J = 5.0, 1H), 8.01 (d, J = 8.8, 2H), 7.80 (d, J = 9.0, 3H), 7.75 (d, J = 7.5, 1H), 7.59 (m, 2H), 6.90 (d, J = 5.0, 1H), 3.17 (s, 3H), 3.14 (s, 3H), 3.04 (m, 2H) |
| | 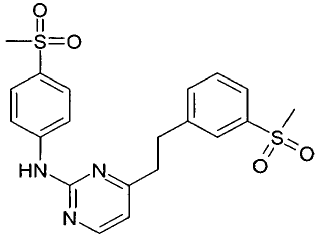 | |
| "A5" | N-Methyl-N-(2-{2-[2-(2-oxo-2,3-dihydro-1H-indol-5-ylamino)-pyrimidin-4-yl]-ethyl}-phenyl)-methansulfonamid | 10.21 (br. s, 1H), 9.37 (br. s, 1H), 8.29 (d, J = 6.0, 1H), 7.65 (m, 1H), 7.53 - 7.51 (m, 1H), 7.46 (m, 1H), 7.37 (m, 1H), 7.31 - 7.29 (m, 2H), 6.72 (d, J = 8.6, 1H), 6.65 (d, J = 6.1, 1H), 3.31 (m, 2H), 3.13 (s, 3H), 3.14 (m, 2H), 3.07 (s, 3H), 2.89 (m, 2H). |
| | 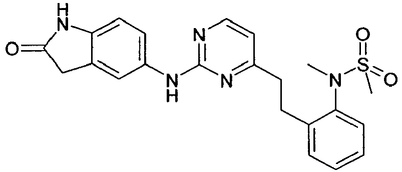 | |
| "A6" | N-Methyl-N-(3-{2-[2-(2-oxo-2,3-dihydro-1H-indol-5-ylamino)-pyrimidin-4-yl]-ethyl}-pyridin-2-yl)-methansulfonamid | 10.21 (br. s, 1H), 9.40 (br. s, 1H), 8.39 (dd, J = 1.6, J = 4.4, 1H), 8.29 (d, J = 6.0, 1H), 7.84 (dd, J = 1.6, J = 7.6, 1H), 7.64 (m, 1H), 7.51 (m, 1H), 7.38 (dd, J = 4.8, J = 7.6 1H), 6.71 (d, J = 8.6, 1H), 6.64 (d, J = 6.1, 1H), 3.45 (m, 2H), 3.14 (s, 3H), 3.13 - 3.12 (m, 2H), 3.11 (s, 3H), 2.92 (m, 2H) |
| | 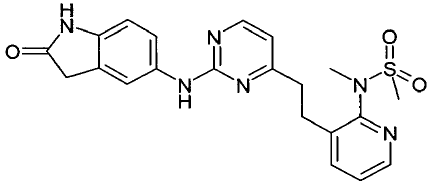 | |
| "A7" | N-Methyl-N-(3-{2-[2-(2-oxo-1,2,3,4-tetrahydro-chinolin-6-ylamino)-pyrimidin-4-yl]-ethyl}-pyridin-2-yl)-methansulfonamid | 9.93 (s, 1H), 9.40 (s, 1H), 8.38 (dd, J = 4.7, 1.8, 1H), 8.29 (d, J = 5.0, 1H), 7.84 (dd, J = 7.7, 1.6, 1H), 7.56 (d, J = 2.0, 1H), 7.47 (dd, J = 8.4, 2.3, 1H), 7.38 (dd, J = 7.7,4.7, 1H), 6.75 (d, J = 8.5, 1H), 6.65 (d, J = 5.0, 1H), 3.13 (s, 3H), 3.10 (s, 3H), 2.96 - 2.90 (m, 2H), 2.85 - 2.80 (m, 2H), 2.44 - 2.37 (m, 4H) |
| | 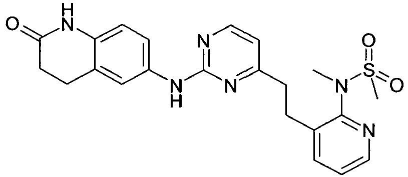 | |
| "A8" | N-(3-{2-[2-(4-Methansulfonyl-phenylamino)-pyrimidin-4-yl]-ethyl}-pyridin-2-yl)-N-methyl-methansulfonamid | 10.17 (s, 1H), 8.42 (d, J = 5.0, 2H), 8.01 (m, 2H), 7.79 (d, J = 8.9, 2H), 7.86 (m, 1H), 7.80 (m, 2H), 7.40 (s, 1H), 6.86 (s, 1H), 3.33 (s, 3H), 3.15 - 3.12 (m, 5H), 3.01 (m, 2H) |
| | 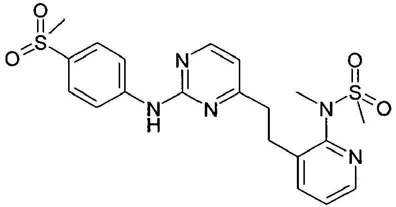 | |
| "A9" | (2-{2-[2-(2-Oxo-2,3-dihydro-1H-indol-5-ylamino)-pyrimidin-4-yl]-ethyl}-phenyl)-acetonitril | 10.18 (s, 1 H), 9.35 (s, 1 H), 8.30 (d, 1 H), 7.66 (d, 1 H), 7.53 (dd, 1 H), 7.34 - 7.41 (m, 1 H), 7.18 - 7.32 (m, 3 H), 6.73 (d, 1 H), 6.70 (d, 1 H), 4.07 (s, 2 H), 3.46 (s, 2 H), 2.98 - 3.11 (m, 2 H), 2.82 - 2.97 (m, 2 H) |
| | 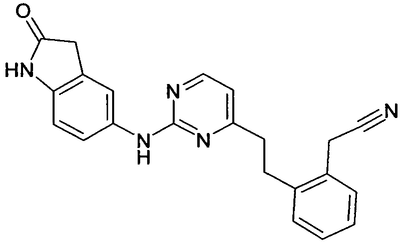 | |
| "A10" | N-Methyl-N-(3-{2-[2-(2-oxo-2,3-dihydro-1H-indol-5-ylamino)-pyrimidin-4-yl]-ethyl}-pyridin-2-yl)-acetamid | 10.18 (s, 1 H), 9.32 (s, 1 H), 8.40 (dd, 1H), 8.29 (d, 1H), 7.95 and 7.74 (dd,1H), 7.60 (d,1H), 7.49 (dd,1H), 7.29 and 7.42 (dd, 1H), 6.73 (d, 1H), 6.68 (d,1H), 3.46 (s,2H),3.05 and 3.22 (s,3H), 2.87-3.03 ( m,4H), 1.61 and 2.17 (s,3H) |
| | 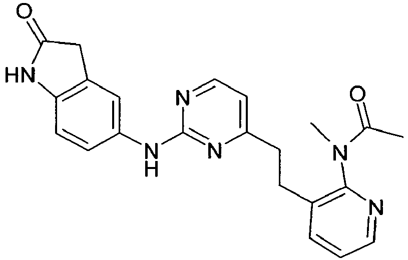 | |
| "A11" | (4-Fluor-2-{2-[2-(2-oxo-2,3-dihydro-1H-indol-5-ylamino)-pyrimidin-4-yl]-ethyl}-phenyl)-acetonitril | 10.19 (s, 1 H) 9.35 (s, 1 H) 8.31 (d, 1 H) 7.65 (s,1 H) 7.33 - 7.57 (m, 2H) 6.95 - 7.27 (m, 2 H) 6.58 - 6.83 (m, 2 H) 4.05 (s, 2 H) 3.46 (s, 2 H) 2.99 - 3.16 (m, 2 H) 2.80 - 2.99 (m, 2 H) |
| | 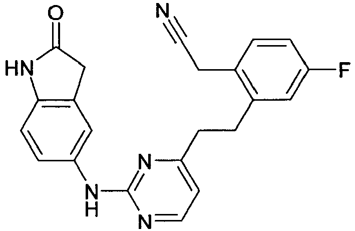 | |
| "A12" | N-Methyl-N-(3-{2-[2-(2-oxo-2,3-dihydro-1H-indol-5-ylamino)-pyrimidin-4-yl]-ethyl}-pyridin-2-yl)-formamid | 10.18 (s, 1 H) 9.18 - 9.43 (m, 1 H) 8.17 - 8.48 (m, 3 H) 7.75 - 8.01 (m, 1H) 7.55 - 7.71 (m, 1 H) 7.42 - 7.55 (m, 1 H) 7.23 - 7.42 (m, 1 H) 6.52 - 6.86 (m, 2 H) 3.46 (s, 2 H) 3.14 (s, 3H), 2.96 - 3.10 (m, 2 H) 2.82 - 2.96 (m, 2 H) |
| | 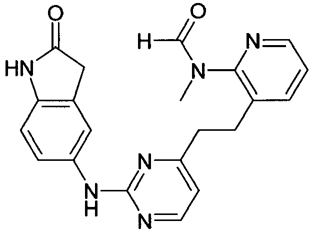 | |
| "A13" | 1-(2-{2-[2-(2-Oxo-2,3-dihydro-1H-indol-5-ylamino)-pyrimidin-4-yl]-ethyl}-phenyl)-cyclobutancarbonitril | 10.19 (s, 1 H) 9.34 (s, 1 H) 8.33 (d, 1 H) 7.50 - 7.71 (m, 2 H) 7.14 - 7.47 (m, 4 H) 6.77 (s, 1 H) 6.73 (d, 1 H) 3.46 (s, 2 H) 2.87 - 3.09 (m, 4 H) 2.59 - 2.87 (m, 4 H) 2.23 - 2.45 (m, 1 H) 1.83 - 2.02 (m, 1 H) |
| | 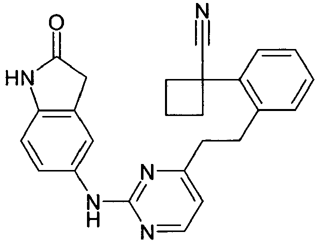 | |
| "A14" | N-[2-(3-{2-[2-(2-Oxo-2,3-dihydro-1H-indol-5-ylamino)-pyrimidin-4-yl]-ethyl}-pyridin-2-ylamino)-ethyl]-formamid | 10.18 (s, 1 H) 9.31 (s, 1 H) 8.30 (d, 1 H) 8.05 - 8.10 (m, 1 H) 8.05 (s, 1H) 7.88 (dd, 1 H) 7.63 (d, 1 H) 7.51 (dd, 1 H) 7.20 (dd, 1 H) 6.74 (d, 1 H) 6.71 (d, 1 H) 6.47 (dd, 1 H) 6.07 -6.21 (m, 1 H) 3.45 (s, 2 H) 3.38 - 3.44 (m, 2 H) 3.31 - 3.37 (m, 2 H) 2.78 - 2.99 (m, 4 H) |
| | 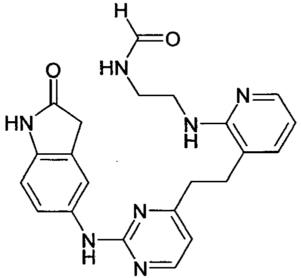 | |
| "A15" | (2-{2-[2-(2-Oxo-2,3-dihydro-1H-indol-5-ylamino)-pyrimidin-4-yl]-ethyl}-5-trifluormethyl-phenyl)-acetonitril | 10.19 (s, 1 H), 9.35 (s, 1 H), 8.31 (d, 1 H), 7.75 (s, 1 H), 7.59 - 7.71 (m,2 H), 7.42 - 7.58 (m, 2 H), 6.73 (d, 1 H), 6.71 (d, 1 H), 4.21 (s, 2 H), 3.46 (s, 2 H), 3.09 - 3.20 (m, 2 H), 2.87 -3.02 (m, 2 H) |
| | 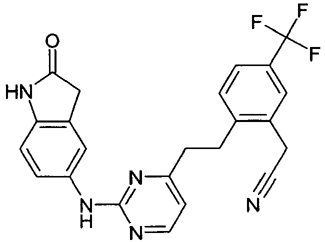 | |
| "A17" | (2-{2-[2-(2-Oxo-2,3-dihydro-1H-indol-5-ylamino)-pyrimidin-4-yl]-ethyl}-4-trifluormethyl-phenyl)-acetonitril | 10.19 (s, 1 H) 9.34 (s, 1 H) 8.30 (d, 1 H) 7.58 - 7.72 (m, 4 H) 7.52 (dd, 1H) 6.73 (d, 1 H) 6.70 (d, 1 H) 4.22 (s, 2 H) 3.46 (s, 2 H) 3.06 - 3.21 (m, 2 H) 2.87 - 3.04 (m, 2 H) |
| | 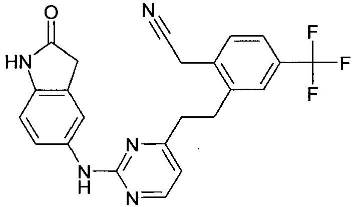 | |
| "A20" | N-Methyl-N-(3-{2-[2-(2-oxo-2,3-dihydro-benzoxazol-6-ylamino)-pyrimidin-4-yl]-ethyl}-pyridin-2-yl)-methanesulfonamid | 11.40 (s, 1H), 9.61 (s, 1H), 8.39 (dd, J = 4.7, 1.8, 1H), 8.35 (d, J = 5.0, 1H), 7.85 (dt, J = 4.5, 2.4, 2H), 7.45 (dd, J = 8.5, 2.0, 1H), 7.39 (dd, J = 7.7, 4.7, 1H), 7.00 (d, J = 8.5, 1H), 6.72 (d, J = 5.0, 1H), 3.18 (m, 2H), 3.15 (s, 4H), 3.13 (s, 3H), 3.01 - 2.93 (m, 2H) |
| | 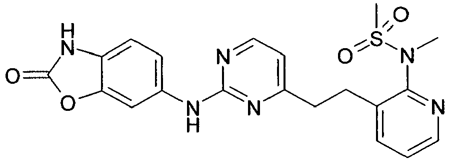 | |
| "A21 " | N-Methyl-N-(3-{2-[2-(1-methyl-2-oxo-2,3-dihydro-1H-indol-5-ylamino)-pyrimidin-4-yl]-ethyl}-pyridin-2-yl)-methanesulfonamid | 9.45 (s, 1H), 8.39 (dd, J = 4.7, 1.8, 1H), 8.30 (d, J = 5.0, 1H), 7.84 (dd, J = 7.7, 1.8, 1H), 7.70 (d, J = 1.8, 1H), 7.60 (dd, J = 8.4, 2.1, 1H), 7.38 (dd, J = 7.7, 4.7, 1H), 6.88 (d, J = 8.5, 1H), 6.67 (d, J = 5.0, 1H), 3.16 (m, 2H), 3.14 (s, 3H), 3.12 (s, 3H), 3.10 (s, 3H), 2.98 - 2.92 (m, 2H) |
| | 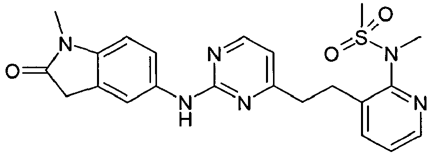 | |
| "A22" | N-(3-{2-[2-(3-Cyan-phenylamino)-pyrimidin-4-yl]-ethyl}-pyridin-2-yl)-N-methyl-methansulfonamid | HPLC-MS; rt; [M+H⁺]* 2.082 [409.2] * |
| | 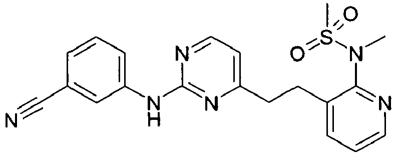 | |
| "A23" | N-(3-{2-[2-(3-Cyan-4-fluor-phenylamino)-pyrimidin-4-yl]-ethyl}-pyridin-2-yl)-N-methyl-methansulfonamid | HPLC-MS; rt; [M+H⁺]* 2.155 [427.2] * |
| | 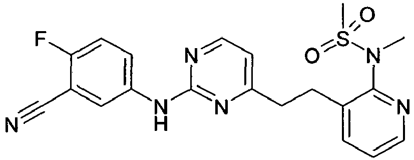 | |
| "A24" | N-Methyl-N-[3-(2-{2-[(6-oxo-1,6-dihydro-pyridazin-3-ylmethyl)-amino]-pyrimidin-4-yl}-ethyl)-pyridin-2-yl]-methansulfonamid | 12.80 (s, 1H), 8.37 (dd, J = 4.7, 1.8, 1H), 8.18 (d, J = 5.1, 1H), 7.75 (br. m, 2H), 7.37 (d, J = 9.7, 1H), 7.37 - 7.32 (m, 1H), 6.83 (d, J = 9.7, 1H), 6.54 (d, J = 5.1, 1H), 4.38 (s, 2H), 3.13 (s, 3H), 3.10 (s, 3H), 3.08 (m, 2H), 2.90 - 2.80 (m, 2H) |
| | 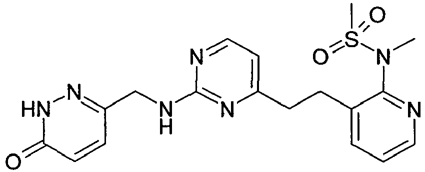 | |
| "A25" | N-(3-{2-[2-(1,3-Dimethyl-2-oxo-2,3-dihydro-1H-benzimidazol-5-ylamino)-pyrimidin-4-yl]-ethyl}-pyridin-2-yl)-N-methyl-methansulfonamid | 9.48 (br. s, 1H), 8.38 (dd, J = 4.7, 1.9, 1H), 8.31 (d, J = 5.0, 1H), 7.85 (dd, J = 7.7, 1.8, 1H), 7.73 (d, J = 1.9, 1H), 7.38 (dd, J = 7.7, 4.7, 1H), 7.33 (dd, J = 8.4, 2.0, 1H), 7.03 (d, J = 8.4, 1H), 6.66 (d, J = 5.0, 1H), 3.30 (s, 3H), 3.29 (s, 3H), 3.21 - 3.14 (m, 2H), 3.13 (s, 3H), 3.10 (s, 3H), 2.96 (m, 2H) |
| | 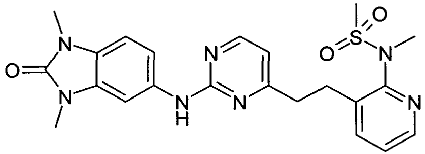 | |
| "A26" | N-(3-{[5-Cyan-2-(2-oxo-2,3-dihydro-benzoxazol-6-ylamino)-pyrimidin-4-ylamino]-methyl}-pyridin-2-yl)-N-methyl-methansulfonamid | HPLC-MS; rt; [M+H⁺]* 1.759 [467.2] * |
| | 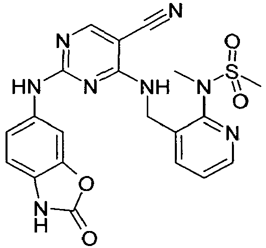 | |
| "A27" | N-Methyl-N-(2-{2-[2-(2-oxo-2,3-dihydro-benzoxazol-6-ylamino)-pyrimidin-4-yl]-ethyl}-phenyl)-methansulfonamid | HPLC-MS; rt; [M+H⁺]* 1.854 [440.2] * |
| | 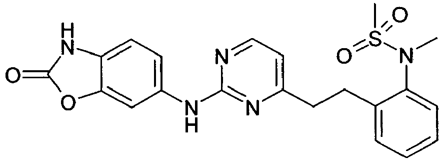 | |
| "A28" | N-(2-{2-[2-(3-Cyan-phenylamino)-pyrimidin-4-yl]-ethyl}-phenyl)-N-methyl-methansulfonamid | HPLC-MS; rt; [M+H⁺]* 2.225 [408.2] * |
| | 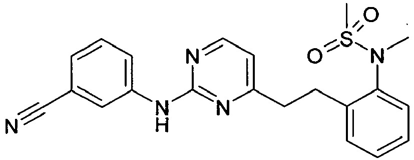 | |
| "A29" | N-(2-{2-[2-(3-Cyan-4-fluor-phenylamino)-pyrimidin-4-yl]-ethyl}phenyl)-N-methyl-methansulfonamid | HPLC-MS; rt; [M+H⁺]* 2.280 [426.2] * |
| | 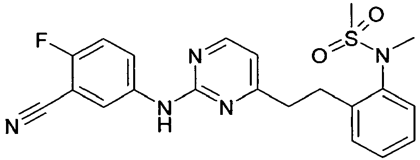 | |
| "A30" | N-Methyl-N-(2-{2-[2-(2-oxo-2,3-dihydro-benzothiazol-5-ylamino)-pyrimidin-4-yl]-ethyl}-phenyl)-methansulfonamid | § 8.41 (d, J = 6.0, 1H), 7.81 (d, J = 1.8, 1H), 7.53 - 7.40 (m, 3H), 7.38 - 7.31 (m, 2H), 7.23 (d, J = 8.6, 1H), 7.04 (d, J = 6.1, 1H), 3.31 (m, 2H), 3.19 (s, 3H), 3.14 (m, 2H), 3.08 (s, 3H) |
| | 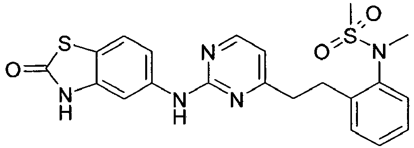 | |
| "A31" | N-(2-{2-[2-(1-Acetyl-2,3-dihydro-1H-indol-5-ylamino)-pyrimidin-4-yl]-ethyl}-phenyl)-N-methyl-methansulfonamid | § 8.39 (d, J = 6.0, 1H), 8.10 (d, J = 8.6, 1H), 7.54 - 7.46 (m, 2H), 7.42 (dd, J = 5.9, 3.5, 1H), 7.38 - 7.27 (m, 3H), 7.00 (d, J = 6.0, 1H), 4.14 (m, 2H), 3.21 (m, 5H), 3.21 (m, 2H), 3.12 (s, 3H), 3.08 (s, 3H), 2.18 (s, 3H) |
| | 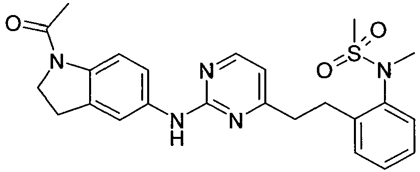 | |
| "A32" | 2-[3-(4-{2-[2-(Methansulfonyl-methyl-amino)-phenyl]-ethyl}-pyrimidin-2-ylamino)-phenyl]-N-methyl-acetamid | 9.44 (br. s, 1H), 8.33 (d, J = 5.0, 1H), 7.82 (br. d, J = 4.6, 1H), 7.67 (d, J = 8.6, 2H), 7.51 - 7.45 (m, 1H), 7.42 - 7.36 (m, 1H), 7.36 - 7.23 (m, 2H), 7.14 (d, J = 8.6, 2H), 6.70 (d, J = 5.0, 1H), 3.15 (s, 3H), 3.08 (s, 3H), 2.93 (m, 2H), 2.59 (s, 1H), 2.58 (d, J = 4.6, 3H) |
| | 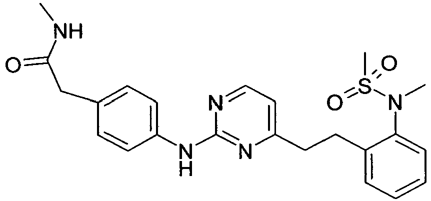 | |
| "A33" | N-Methyl-N-[2-(2-{2-[4-(3-oxo-morpholin-4-yl)-phenylamino]-pyrimidin-4-yl}-ethyl)-phenyl]-methansulfonamid | § 8.48 (d, J = 5.9, 1H), 7.68 (d, J = 8.7, 2H), 7.61 - 7.55 (m, 1H), 7.51 (m, 1H), 7.47 - 7.39 (m, 3H), 7.39 - 7.29 (m, 2H), 7.06 (d, J = 5.9, 1H), 4.20 (m 3H), 4.08 - 3.97 (m, 3H), 3.78 (dd, J = 9.2, 4.1, 3H), 3.33 (d, J = 9.7, 1H), 3.20 (s, 3H), 3.19 - 3.11 (m, 4H), 3.07 (s 3H) |
| | 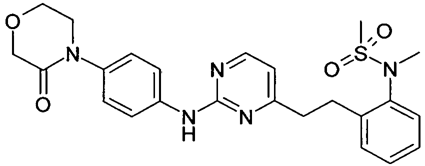 | |
| "A34" | N-[4-(4-{2-[2-(Methansulfonyl-methyl-amino)-phenyl]-ethyl}-pyrimidin-2-ylamino)-phenyl]-nicotinamid | 10.31 (br. s, 1H), 9.51 (s, 1H), 9.10 (d, J = 1.7, 1H), 8.75 (dd, J = 4.8, 1.6, 1H), 8.34 (d, J = 5.0, 1H), 8.32 - 8.24 (m, 1H), 7.79 - 7.72 (m, 2H), 7.64 (d, J = 9.0, 2H), 7.60 - 7.52 (m, 1H), 7.47 (m, 1H), 7.43 - 7.36 (m, 1H), 7.36 - 7.22 (m, 2H), 6.70 (d, J = 5.0, 1H), 3.15 (s, 3H), 3.07 (s, 3H), 3.05 (m, 2H), 2.94 (m, 2H) |
| | 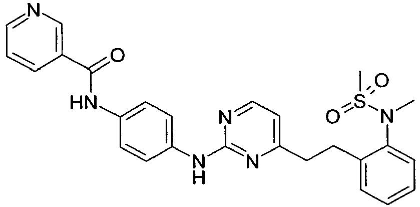 | |
| "A35" | N-Methyl-N-[2-(2-{2-[4-(2-oxo-pyrrolidin-1-yl)-phenylamino]-pyrimidin-4-yl}-ethyl)-phenyl]-methansulfonamid | § 8.35 (d, J = 5.9, 1H), 7.67 (d, J = 9.0, 2H), 7.51 (d, J = 8.9, 2H), 7.41 (m, 1H), 7.35 (dd, J = 6.1, 3.2, 1H), 7.32 - 7.17 (m, 2H), 6.99 (d, J = 6.2, 1H), 3.81 (m, 2H), 3.28 (m, 2H), 3.12 (s, 3H), 3.08 (m, 2H), 2.99 (s, 3H), 2.46 (m, 2H), 2.12 -1.95 (m, 2H) |
| | 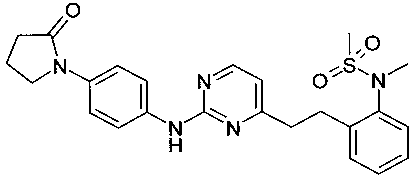 | |
| "A36" | N-[2-(2-{2-[3,5-Dichlor-4-(2-oxo-pyrrolidin-1-yl)-phenylamino]-pyrimidin-4-yl}-ethyl)-phenyl]-N-methyl-methansulfonamid | § 8.48 (d, J = 5.2, 1H), 8.08 (s, 2H), 7.48 (m, 1H), 7.44 - 7.39 (m, 1H), 7.37 - 7.27 (m, 2H), 6.92 (d, J = 5.2, 1H), 3.65 (t, J = 6.9, 2H), 3.33 (m, 2H), 3.20 (s, 3H), 3.11 (m, 2H), 3.08 (s, 3H), 2.46 (t, J = 8.0, 2H), 2.29 - 2.14 (m, 2H) |
| | 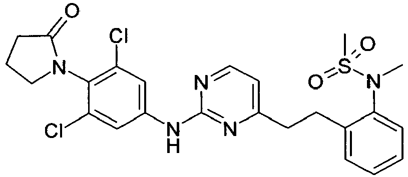 | |
| "A37" | N-[2-(2-{2-[3-Cyan-4-(2-oxo-piperidin-1-yl)-phenylamino]-pyrimidin-4-yl}-ethyl)-phenyl]-N-methyl-methansulfonamid | 10.01 (s, 1H), 8.43 (d, J = 5.0, 1H), 8.26 (d, J = 2.5, 1H), 8.06 (dd, J = 8.9, 2.5, 1H), 7.50 - 7.45 (m, 1H), 7.41 - 7.37 (m, 2H), 7.34 - 7.27 (m, 2H), 6.81 (dd, J = 15.9, 5.0, 1H), 3.61 - 3.50 (m, 2H), 3.30 - 3.21 (m, 3H), 3.13 (s, 3H), 3.07 (s, 3H), 3.06 - 2.88 (m, 3H), 2.41 (m, 2H), 1.95 - 1.78 (m, 4H) |
| | 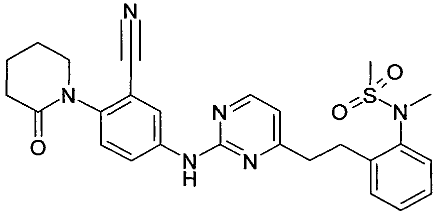 | |
| "A38" | N-[2-(2-{2-[4-(1,3-Dioxo-1,3-dihydro-isoindol-2-yl)-phenylamino]-pyrimidin-4-yl}-ethyl)-phenyl]-N-methyl-methansulfonamid | § 8.51 (d, J = 5.8, 1H), 8.04 - 7.95 (m, 2H), 7.95 - 7.87 (m, 2H), 7.83 (d, J = 11.1, 2H), 7.51 (dd, J = 9.2, 2.5, 3H), 7.48 - 7.41 (m, 1H), 7.41 - 7.27 (m, 2H), 7.07 (d, J = 5.9, 1H), 3.44 - 3.28 (m, 1H), 3.20 (s, 3H), 3.19 - 3.11 (m, 3H), 3.09 (s, 3H) |
| | 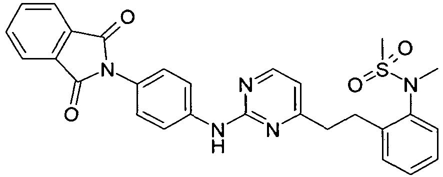 | |
| "A39" | N-Methyl-N-[3-(2-{2-[4-(2-oxo-pyrrolidin-1-yl)-phenylamino]-pyrimidin-4-yl}-ethyl)-pyridin-2-yl]-methansulfonamid | 9.52 (s, 1H), 8.40 (dd, J = 4.7, 1.8, 1H), 8.34 (d, J = 5.5, 1H), 7.85 (dd, J = 7.7, 1.8, 1H), 7.79 - 7.69 (m, 2H), 7.60 - 7.47 (m, 2H), 7.40 (dd, J = 7.7, 4.7, 1H), 6.70 (d, J = 5.0, 1H), 3.81 (t, J = 7.0, 2H), 3.18 (m, 2H), 3.15 (s, 3H), 3.13 (s, 3H), 3.02 - 2.85 (m, 2H), 2.50 - 2.39 (m, 2H), 2.15 - 1.97 (m, 2H) |
| | 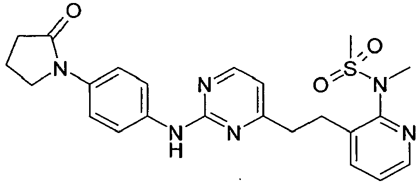 | |
| "A40" | N-[4-(4-{2-[2-(Methansulfonyl-methyl-amino)-pyridin-3-yl]-ethyl}-pyrimidin-2-ylamino)-phenyl]-nicotinamid | 9.44 (d, J = 1.7, 1H), 9.12 - 9.02 (m, 2H), 8.42 - 8.32 (m, 2H), 8.18 (dd, J = 7.9, 6.0, 1H), 7.86 - 7.77 (m, 3H), 7.57 (d, J = 8.8, 2H), 7.31 (dd, J = 7.6, 4.8, 1H), 6.98 (d, J = 6.1, 1H), 3.23 - 3.16 (m, 2H), 3.14 (m, 2H), 3.12 (s, 3H), 3.08 (s, 3H) |
| | 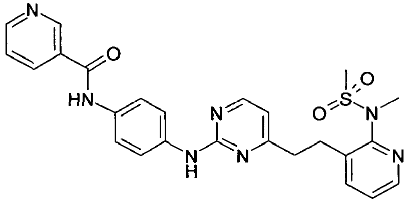 | |
| "A41" | N-Methyl-N-[3-(2-{2-[4-(3-oxo-morpholin-4-yl)-phenylamino]-pyrimidin-4-yl}-ethyl)-pyridin-2-yl]-methansulfonamid | § 8.38 (dd, J = 18.5, 5.3, 2H), 7.84 (d, J = 7.6, 1H), 7.60 (d, J = 7.5, 1H), 7.42 - 7.37 (m, 1H), 7.36-7.32 (m, 1H), 6.98 (d, J = 6.0, 1H), 4.18 (s, 1H), 3.97 - 3.93 (m, 2H), 3.76 - 3.67 (m, 1H), 3.19 (m, 1H), 3.13 (m, 2H), 3.11 (s, 3H), 3.10 (s, 3H) |
| | 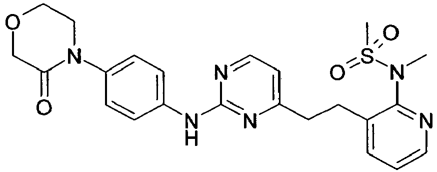 | |
| "A42" | N-[3-(2-{2-[3-Cyan-4-(2-oxo-piperidin-1-yl)-phenylamino]-pyrimidin-4-yl}-ethyl)-pyridin-2-yl]-N-methyl-methansulfonamid | 10.02 (s, 1H), 8.43 (d, J = 5.0, 1H), 8.42 - 8.37 (m, 1H), 8.25 (d, J = 2.5, 1H), 8.06 (dd, J = 8.9, 2.5, 1H), 7.86 (dd, J = 7.7, 1.8, 1H), 7.41 (s, 1H), 7.39 (dd, J = 4.6, 3.0, 1H), 6.83 (d, J = 5.0, 1H), 3.57 (m, 2H), 3.50 - 3.30 (m, 2H), 3.20 - 3.15 (m, 2H), 3.15 (s, 3H), 3.12 (s, 3H), 3.04 - 2.98 (m, 2H), 2.42 (m, 2H), 1.96 - 1.82 (m, 4H) |
| | 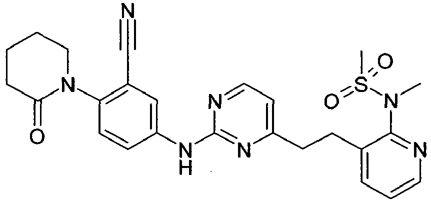 | |
| "A43" | N-[3-(2-{2-[4-(1,3-Dioxo-1,3-dihydro-isoindol-2-yl)-phenylamino]-pyrimidin-4-yl}-ethyl)-pyridin-2-yl]-N-methyl-methansulfonamid | 9.78 (s, 1H), 8.39 (d, J = 5.0, 2H), 7.98 - 7.94 (m, 2H), 7.93 - 7.84 (m, 5H), 7.39 (dd, J = 7.7, 4.7, 1H), 7.34 - 7.29 (m, 2H), 6.76 (d, J = 5.0, 1H), 3.22 - 3.16 (m, 2H), 3.14 (s, 3H), 3.12 (s, 3H), 2.99 (m, 2H) |
| | 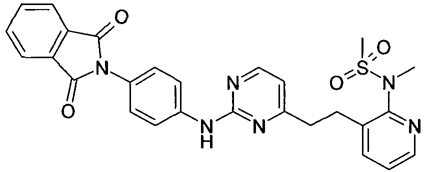 | |
| "A44" | N-Methyl-N-(5-methyl-3-{2-[2-(2-oxo-2,3-dihydro-1H-indol-5-ylamino)-pyrimidin-4-yl]-ethyl}-pyridin-2-yl)-methansulfonamid | 10.18 (s, 1 H), 9.36 (s, 1 H), 8.30 (d, 1 H), 8.21 (s, 1 H), 7.59 - 7.78 (m, 2 H), 7.52 (d, 1 H), 6.73 (d, 1 H), 6.65 (d, 1 H), 3.45 (br. s., 2 H), 3.11 (s, 3 H), 3.09 (s, 3 H), 3.06 - 3.16 (m, 2 H), 2.91 (t, 2 H), 2.29 (s, 3 H) |
| | 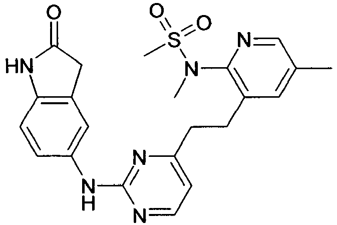 | |
| "A45" | 1-(4-Fluor-2-{2-[2-(2-oxo-2,3-dihydro-1H-indol-5-ylamino)-pyrimidin-4-yl]-ethyl}-phenyl)-cyclobutancarbonitril | 10.19 (s, 1 H) 9.33 (s, 1 H) 8.33 (d, 1 H) 7.56 - 7.64 (m, 2 H) 7.31 (dd, 1H) 7.25 (dd, 1 H) 7.11 (td, 1 H) 6.75 (d, 1 H) 6.74 (d, 1 H) 3.45 (s, 2 H) 2.90 - 3.14 (m, 4 H) 2.75 - 2.85 (m, 2H) 2.60 - 2.71 (m, 2 H) 2.34 (m, 1 H) 1.93 (m, 1 H) |
| | 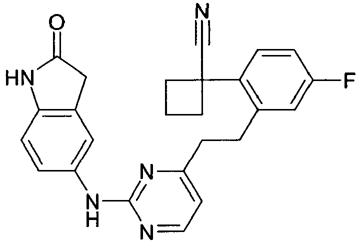 | |
| "A46" | 1-(5-Fluor-2-{2-[2-(2-oxo-2,3-dihydro-1H-indol-5-ylamino)-pyrimidin-4-yl]-ethyl}-phenyl)-cyclobutancarbonitril | 10.19 (s, 1 H), 9.34 (s, 1 H), 8.33 (d, 1 H), 7.62 (d, 1 H), 7.58 (dd, 1 H), 7.41 (dd, 1 H), 7.19 (td, 1 H), 7.12 (dd, 1 H), 6.75 (d, 1 H), 6.73 (d, 1 H), 3.45 (s, 2 H), 2.87 - 3.10 (m, 4 H), 2.61 - 2.87 (m, 4 H), 2.23 - 2.40 (m, 1 H), 1.82 - 2.02 (m, 1 H) |
| | 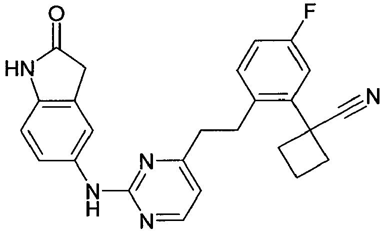 | |
| "A47" | 1-(2-{2-[2-(2-Oxo-2,3-dihydro-1H-indol-5-ylamino)-pyrimidin-4-yl]-ethyl}-5-trifluormethyl-phenyl)-cyclobutancarbonitril | 10.20 (s, 1 H), 9.34 (s, 1 H), 8.34 (d, 1 H), 7.70 - 7.75 (m, 1 H), 7.43 - 7.66 (m, 4 H), 6.75 (d, 1 H), 6.75 (d, 1 H), 3.45 (s, 2 H), 3.06 - 3.19 (m, 2 H), 2.93 - 3.06 (m, 2 H), 2.67 - 2.92 (m, 4 H), 2.29 - 2.43 (m, 1 H), 1.71 - 2.07 (m, 1 H) |
| | 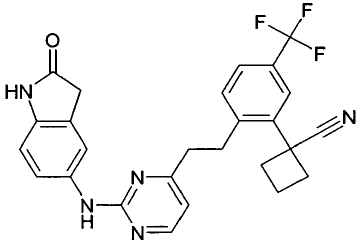 | |
| "A48" | (5-Fluor-4-phenethyl-pyrimidin-2-yl)-phenyl-amin | 10.21 (s, 1H), 8.68 (s, 1H), 7.75 (d, *J* = 7.6, 2H), 7.25 (m, 7H), 7.02 (t, J = 7.4, 1H), 3.06 (m, 4H) |
| | 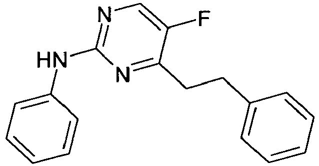 | |
| HPLC-MS; rt; [M+H⁺]* | | |
| RT: 5.62 min; | | |
| Masse gefunden [M]⁺: 294 | | |
| "A49" | (5-Methyl-4-phenethyl-pyrimidin-2-yl)-phenyl-amin | 10.20 (s, 1H), 8.66 (s, 1H), 7.75 (d, *J* = 7.6, 2H), 7.25 (m, 7H), 7.03 (t, J = 7.4, 1H), 3.06 (s, 4H), 2.03 (s, 3H) |
| | 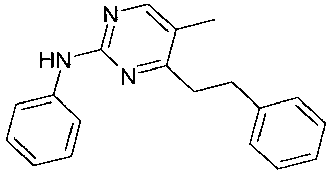 | |
| HPLC-MS; rt; [M+H⁺]* | | |
| RT: 4.43 min; | | |
| Masse gefunden [M]⁺: 290.3 | | |
| "A50" | (4-Phenethyt-5-trifluormethyl-pyrimidin-2-yl)-phenyl-amin | 10.22 (s, 1H), 8.67 (s, 1H), 7.74 (d, J = 7.7, 2H), 7.25 (m, 7H), 7.03 (t, *J* = 7.4, 1H), 3.06 (s, 4H) |
| | 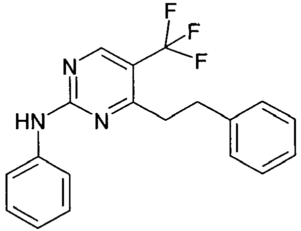 | |
| HPLC-MS; rt; [M+H⁺]* | | |
| RT: 6.14 min; | | |
| Masse gefunden [M]⁺: 344.0 | | |
| "A51" | (5-Nitro-4-phenethyl-pyrimidin-2-yl)-phenyl-amin | |
| | 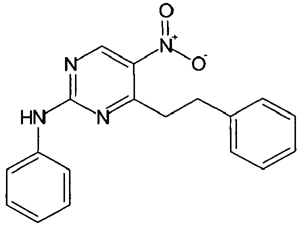 | |
| "A52" | (5-Brom-4-phenethyl-pyrimidin-2-yl)-phenyl-amin | 9.73 (s, 1H), 8.35 (d, *J* = 5.0, 1H), 7.75 (d, *J* = 8.8, 2H), 7.42 (d, *J* = 8.8, 2H), 7.31 - 7.20 (m, 4H), 7.17 (t, J = 6.9, 1H), 6.77 (d, *J* = 5.0, 1H), 3.05 - 2.98 (m, 2H), 2.97 - 2.89 (m, 2H) |
| | 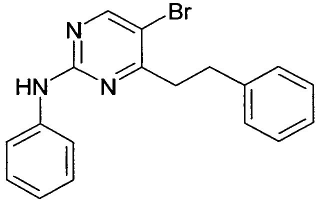 | |
| HPLC-MS; rt; [M+H⁺]* | | |
| RT: 5.27 min; | | |
| Masse gefunden [M]⁺: 356.0 | | |
| "A53" | 5-{5-Fluor-4-[2-(4-fluor-phenyl)-ethyl]-pyrimidin-2-ylamino}-1,3-dihydro-indol-2-on | |
| | 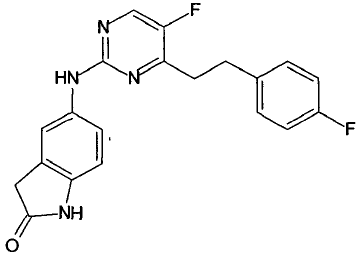 | |
| "A54" | 5-{4-[2-(4-Brom-2-fluor-phenyl)-ethyl]-5-fluor-pyrimidin-2-ylamino}-1,3-dihydro-indol-2-on | |
| | 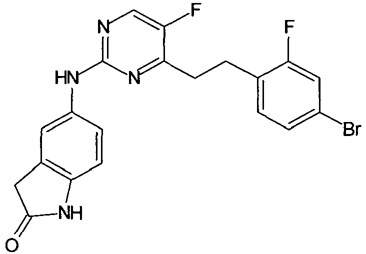 | |
| "A55" | 5-(5-Fluor-4-{2-[2-(propan-1-sulfonyl)-phenyl]-ethyl]-pyrimidin-2-ylamino}-1,3-dihydro-indol-2-on | |
| | 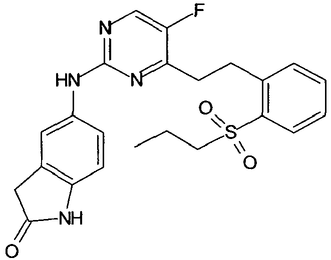 | |
| "A56" | 6-{5-Fluor-4-[2-(4-fluor-phenyl)-ethyl]-pyrimidin-2-ylamino}-3,4-dihydro-1H-chinolin-2-on | 9.94 (s, 1H), 9.47 (s, 1H), 8.34 (d, J = 1.7, 1H), 7.51 (s, 1H), 7.42 (dd, *J* = 8.6, 2.2, 1H), 7.25 (dd, J = 8.5, 5.7, 2H), 7.08 (t, *J* = 8.9, 2H), 6.74 (d, J = 8.6, 1H), 3.00 (m, 4H), 2.81 (m, 2H), 2.44 - 2.38 (m, 2H) |
| | 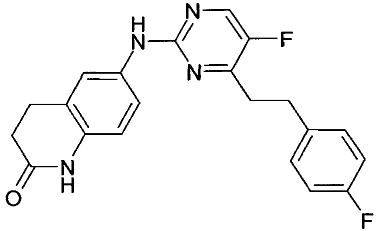 | |
| HPLC-MS; rt; [M+H⁺]* | | |
| RT: 4.53 min; | | |
| Masse gefunden [M]⁺: 381.2 | | |
| "A57" | 6-{4-[2-(4-Brom-2-fluor-phenyl)-ethyl]-5-fluor-pyrimidin-2-ylamino}-3,4-dihydro-1H-chinolin-2-on | |
| | 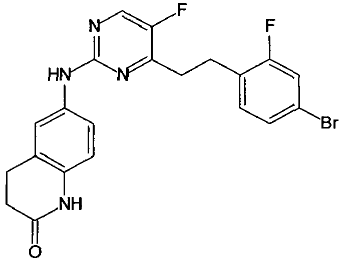 | |
| "A58" | 6-(5-Fluor-4-{2-[2-(propan-1-sulfonyl)-phenyl]-ethyl]-pyrimidin-2-ylamino}-3,4-dihydro-1H-chinolin-2-on | |
| | 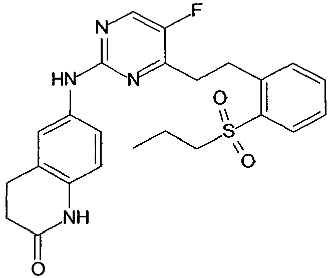 | |
| "A59" | 6-{5-Fluor-4-[2-(4-fluor-phenyl)-ethyl]-pyrimidin-2-ylamino}-3H-benzoxazol-2-on | |
| | 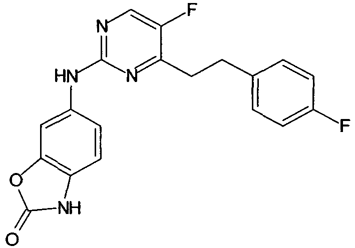 | |
| "A60" | 6-{4-[2-(4-Brom-2-fluor-phenyl)-ethyl]-5-fluor-pyrimidin-2-ylamino}-3H-benzoxazol-2-on | |
| | 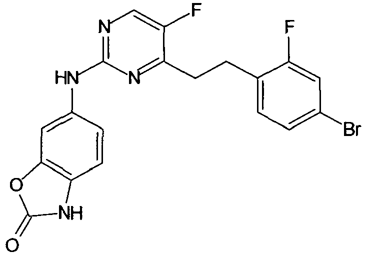 | |
| "A61" | 6-(5-Fluor-4-{2-[2-(propan-1-sulfonyl)-phenyl]-ethyl]-pyrimidin-2-ylamino}-3H-benzoxazol-2-on | |
| | 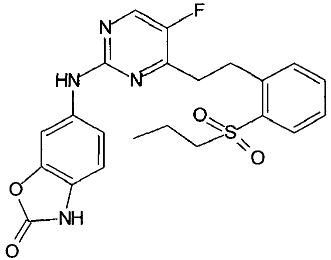 | |
| "A62" | 5-{4-[2-(4-Fluor-phenyl)-ethyl]-5-methyl-pyrimidin-2-ylamino}-1,3-dihydro-indol-2-on | |
| | 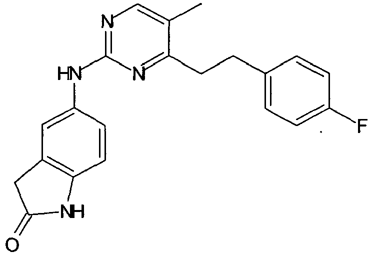 | |
| "A63" | 5-{4-[2-(4-Brom-2-fluor-phenyl)-ethyl]-5-methyl-pyrimidin-2-ylamino}-1,3-dihydro-indol-2-on | |
| | 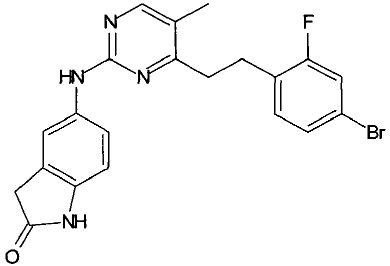 | |
| "A64" | 5-(5-Methyl-4-{2-[2-(propan-1-sulfonyl)-phenyl]-ethyl}-pyrimidin-2-ylamino}-1,3-dihydro-indol-2-on | |
| | 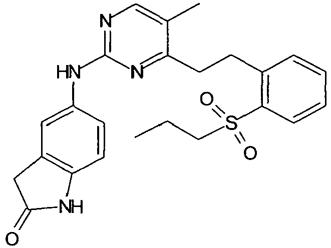 | |
| "A65" | 6-{4-[2-(4-fluor-phenyl)-ethyl]-5-methyl-pyrimidin-2-ylamino}-3,4-dihydro-1H-chinolin-2-on | 9.91 (s, 1H), 9.22 (s, 1H), 8.12 (s, 1H), 7.58 (d, *J* = 2.2, 1H), 7.47 (dd, J = 8.6, 2.4, 1H), 7.27 (dd, J = 8.6, 5.6, 2H), 7.13 - 7.05 (m, 2H), 6.73 (d, *J* = 8.6, 1H), 3.03 - 2.97 (m, 2H), 2.89 (m, 2H), 2.81 (m, 2H), 2.41 (m, 2H), 2.04 (s, 3H) |
| | 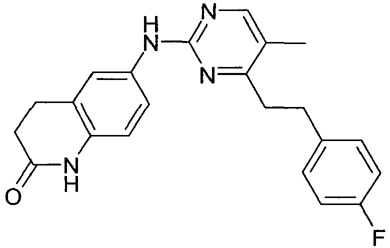 | |
| HPLC-MS; rt; [M+H⁺]* | | |
| RT: 3.55 min; | | |
| Masse gefunden [M]⁺: 377.3 | | |
| "A66" | 6-{4-[2-(4-Brom-2-fluor-phenyl)-ethyl]-5-methyl-pyrimidin-2-ylamino}-3,4-dihydro-1H-chinolin-2-on | |
| | 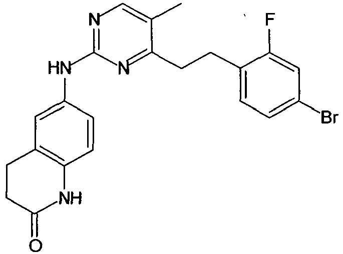 | |
| "A67" | 6-(5-Methyl-4-{2-[2-(propan-1-sulfonyl)-phenyl]-ethyl}-pyrimidin-2-ylamino}-3,4-dihydro-1H-chinolin-2-on | |
| | 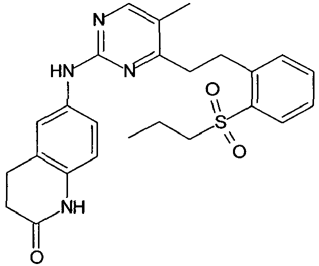 | |
| "A68" | 6-{4-[2-(4-fluor-phenyl)-ethyl]-5-methyl-pyrimidin-2-ylamino}-3H-benzoxazol-2-on | |
| | 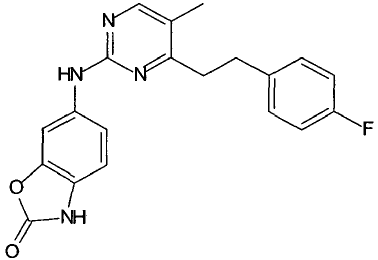 | |
| "A69" | 6-{4-[2-(4-Brom-2-fluor-phenyl)-ethyl]-5-methyl-pyrimidin-2-ylamino}-3H-benzoxazol-2-on | |
| | 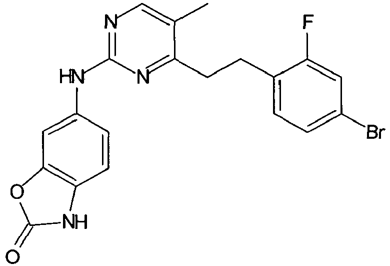 | |
| "A70" | 6-(5-Methyl-4-{2-[2-(propan-1-sulfonyl)-phenyl]-ethyl]-pyrimidin-2-ylamino}-3H-benzoxazol-2-on | |
| | 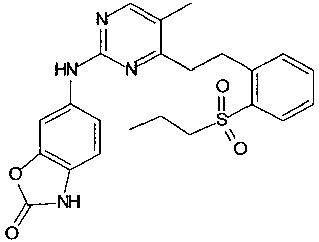 | |
| "A71" | [5-(1-Methyl-1H-pyrazol-4-yl)-4-phenethyl-pyrimidin-2-yl]-phenyl-amin | 9.56 (s, 1H), 8.32 (d, *J* = 5.0, 1H), 8.01 (s, 1H), 7.78 - 7.71 (m, 3H), 7.45 (d, *J* = 8.7, 2H), 7.26 (dd, J = 10.5, 5.8, 4H), 7.17 (t, *J* = 6.8, 1H), 6.72 (d, *J* = 5.0, 1H), 3.84 (s, 3H), 3.06 - 3.00 (m, 2H), 2.96 - 2.89 (m, 2H) |
| | 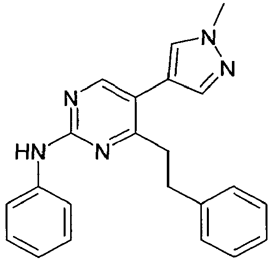 | |
| HPLC-MS; rt; [M+H⁺]* | | |
| RT: 4.05 min; | | |
| Masse gefunden [M]⁺: 356.3 | | |
| "A72" | (5-Furan-2-yl-4-phenethyl-pyrimidin-2-yl]-phenyl-amin | |
| | 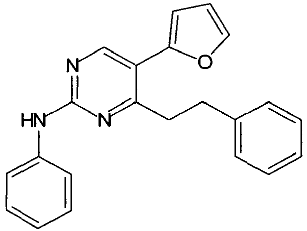 | |
| "A73" | [5-(2-Methyl-thiazol-4-yl)-4-phenethyl-pyrimidin-2-yl]-phenyl-amin | |
| | 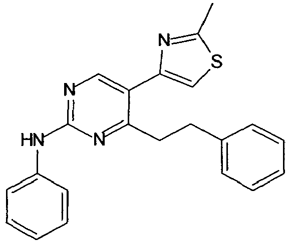 | |
| "A74" | 5-{4-[2-(4-Fluor-phenyl)-ethyl]-5-trifluormethyl-pyrimidin-2-ylamino}-1,3-dihydro-indol-2-on | 10.31 (s, 1H), 10.07 (s, 1H), 8.61 (s, 1H), 7.58 (s, 1H), 7.48 (dd, *J* = 8.4, 2.1, 1H), 7.24 (dd, *J =* 8.2, 5.8, 2H), 7.10 (t, *J =* 8.8, 2H), 6.76 (d, *J* = 8.4, 1H), 3.48 (s, 2H), 3.13 - 2.93 (m, 4H) |
| | 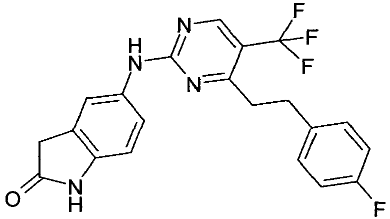 | |
| HPLC-MS; rt; [M+H⁺]* | | |
| RT: 4.99 min; | | |
| Masse gefunden [M]⁺: 417 | | |
| "A75" | 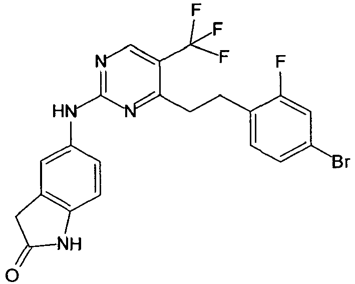 | |
| "A76" | 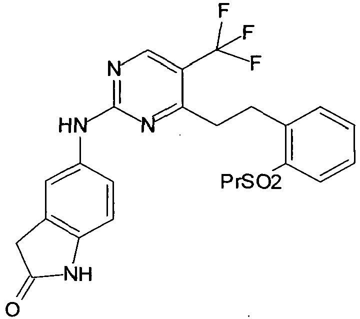 | |
| "A77" | 6-{4-[2-(4-Fluor-phenyl)-ethyl]-5-trifluoromethyl-pyrimidin-2-ylamino}-3,4-dihydro-1H-chinolin-2-on | 10.08 (s, 1H), 10.02 (s, 1H), 8.62 (s, 1H), 7.55 (s, 1H), 7.45 (dd, *J* = 8.6, 2.2, 1H), 7.24 (dd, *J* = 8.4, 5.6, 2H), 7.09 (t, *J =* 8.9, 2H), 6.80 (d, *J* = 8.5, 1H), 3.12 - 2.96 (m, 4H), 2.84 (t, *J* = 7.5, 2H), 2.43 (dd, *J* = 8.4, 6.7, 2H) |
| | 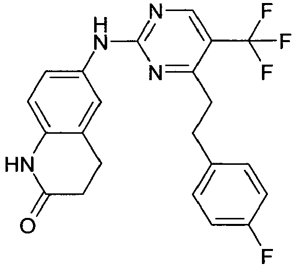 | |
| HPLC-MS; rt; [M+H⁺]* | | |
| RT: 5.11 min; | | |
| Masse gefunden [M]⁺: 431 | | |
| "A78" | 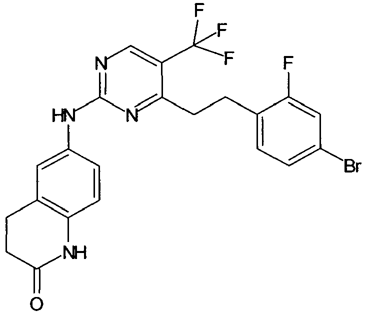 | |
| "A79" | 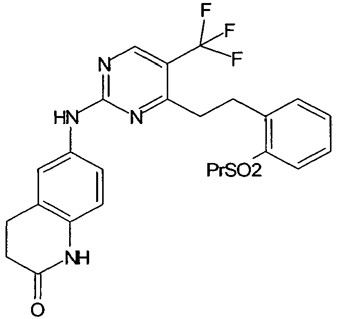 | |
| "A80" | 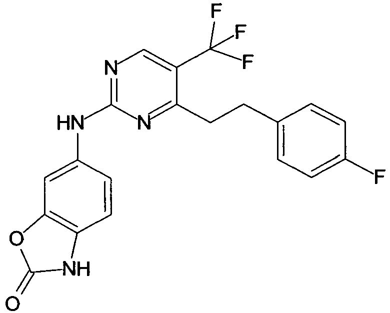 | |
| "A81" | 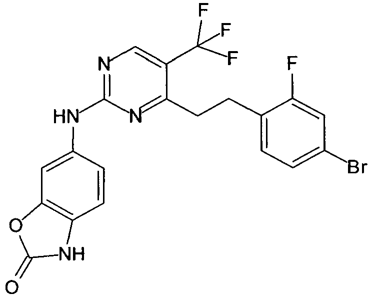 | |
| "A82" | 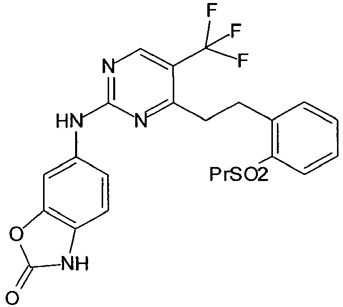 | |
| "A83" | 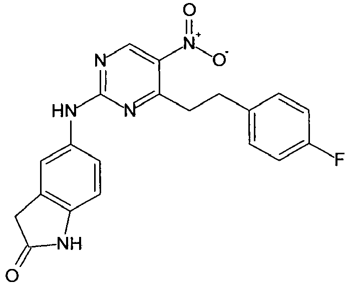 | |
| "A84" | 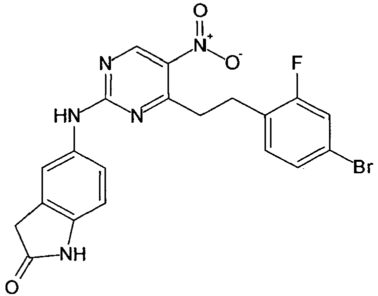 | |
| "A85" | 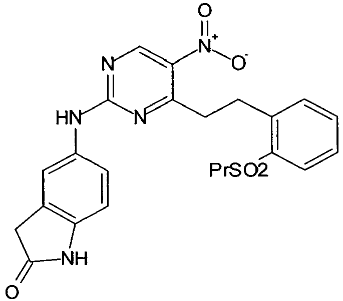 | |
| "A86" | 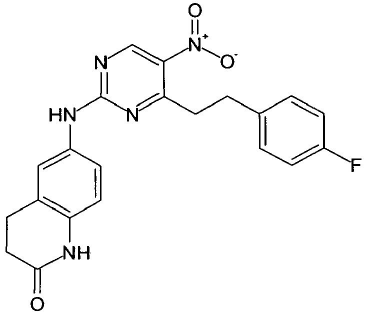 | |
| "A87" | 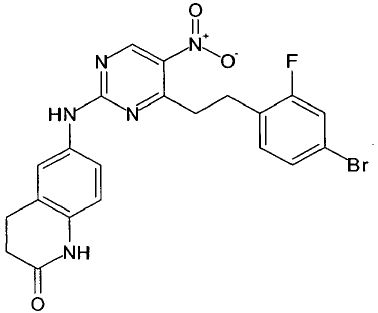 | |
| "A88" | 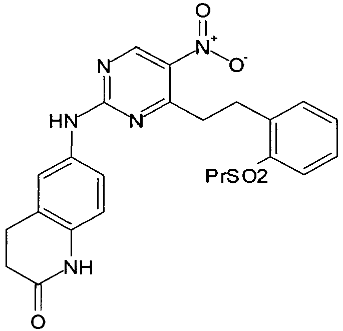 | |
| "A89" | 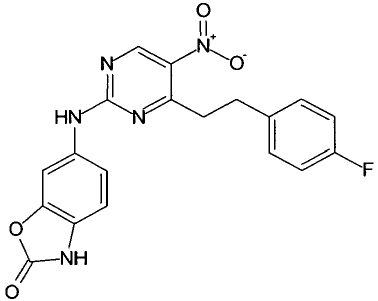 | |
| "A90" | 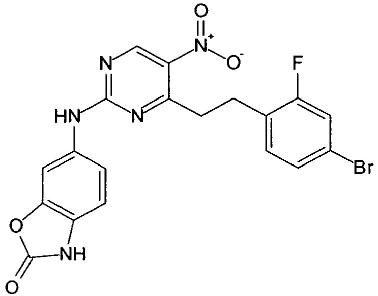 | |
| "A91" | 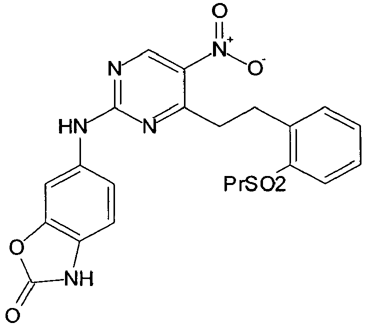 | |
| "A92" | 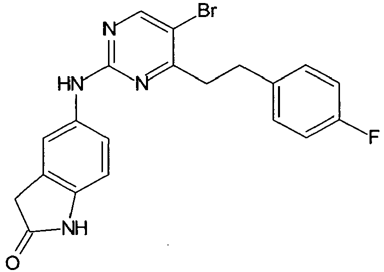 | |
| "A93" | 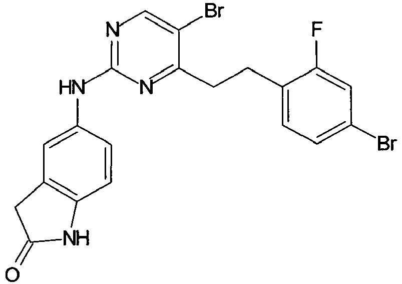 | |
| "A94" | 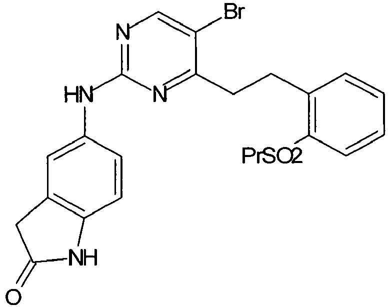 | |
| "A95" | 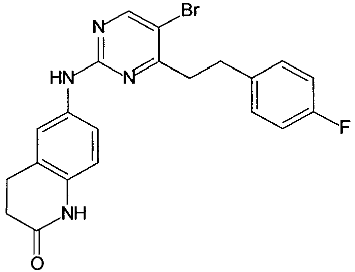 | |
| "A96" | 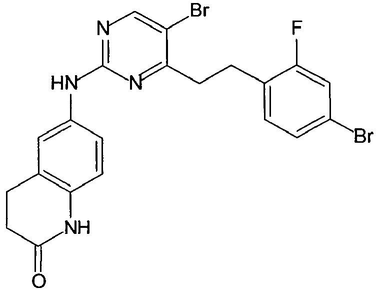 | |
| "A97" | 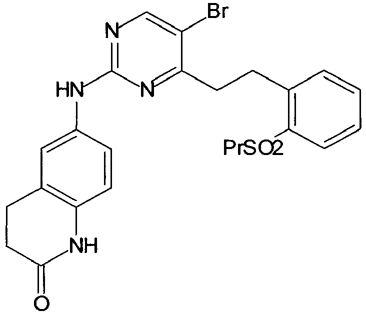 | |
| "A98" | 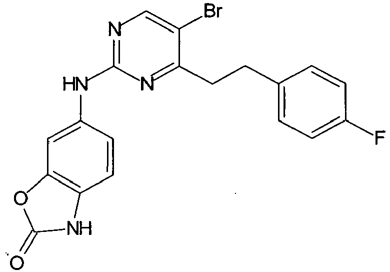 | |
| "A99" | 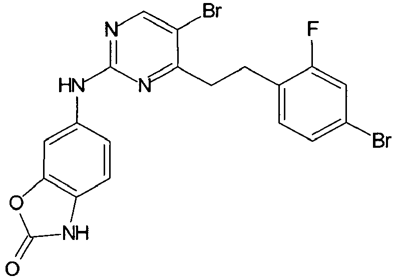 | |
| "A100" | 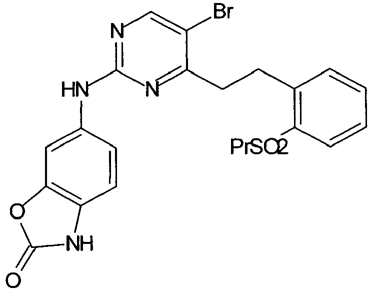 | |
| "A101" | 6-(5-Brom-4-phenethyl-pyrimidin-2-ylamino)-3,4-dihydro-1H-chinolin-2-on | 9.96 (s, 1H), 9.63 (s, 1H), 8.45 (s, 1H), 7.51 (s, 1H), 7.41 (dd, J = 8.6, 2.4, 1H), 7.32 - 7.17 (m, 6H), 6.76 (d, J = 8.5, 1H), 3.02 (s, 4H), 2.85 - 2.79 (m, 2H), 2.44 - 2.39 (m, 2H) |
| | 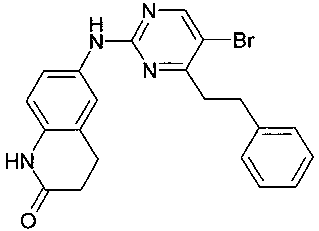 | |
| HPLC-MS; rt; [M+H⁺]* | | |
| RT: 4.88 min; | | |
| Masse gefunden [M]⁺: 423 | | |
| "A102" | 5-(4-Phenethyl-5-trifluormethyl-pyrimidin-2-ylamino)-1,3-dihydro-indol-2-on | 10.31 (s, 1H), 10.07 (s, 1H), 8.61 (s, 1H), 7.58 (s, 1H), 7.48 (d, *J* = 8.6, 1H), 7.29 (t, *J* = 7.4, 2H), 7.20 (dd, *J* = 15.2, 7.1, 3H), 6.76 (d, *J* = 8.4, 1H), 3.48 (s, 2H), 3.03 (s, 4H) |
| | 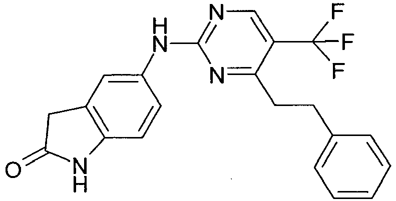 | |
| HPLC-MS; rt; [M+H⁺]* | | |
| RT: 4.94 min; | | |
| Masse gefunden [M]⁺: 399.3 | | |
| "A103" | 6-(4-Phenethyl-5-trifluormethyl-pyrimidin-2-ylamino)-3,4-dihydro-1H-chinolin-2-on | 10.09 (s, 1H), 10.02 (s, 1H), 8.62 (s, 1H), 7.56 (s, 1H), 7.45 (dd, *J* = 8.5, 2.4, 1H), 7.33 - 7.25 (m, 2H), 7.20 (dd, *J* = 16.2, 7.4, 3H), 6.80 (d, *J* = 8.6, 1H), 3.08 - 3.00 (m, 4H), 2.84 (t, *J* = 7.5, 2H), 2.45 - 2.40 (m, 3H) |
| | 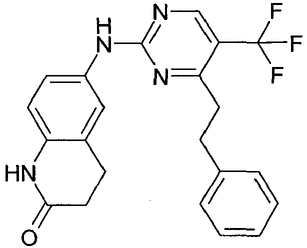 | |
| HPLC-MS; rt; [M+H⁺]* | | |
| RT: 5.17 min; | | |
| Masse gefunden [M]⁺: 413.3 | | |
| "A104" | 6-(5-Methyl-4-{2-[3-(propan-1-sulfonyl)-phenyl]-ethyl}-pyrimidin-2-ylamino)-3,4-dihydro-1H-chinolin-2-on | 9.91 (s, 1H), 9.21 (s, 1H), 8.12 (s, 1H), 7.68 (d, *J* = 7.3, 2H), 7.63 (d, *J* = 7.7, 1H), 7.60-7.52 (m, 2H), 7.45 (dd, *J =* 8.6, 2.3, 1H), 6.73 (d, *J* = 8.6, 1H), 3.22 - 3.11 (m, 4H), 2.97 (t, *J* = 7.6, 2H), 2.81 (t, *J* = 7.5, 2H), 2.44 - 2.38 (m, 2H), 2.03 (s, 3H), 1.45 (m, 2H), 0.85 (t, *J* = 7.4, 3H) |
| | 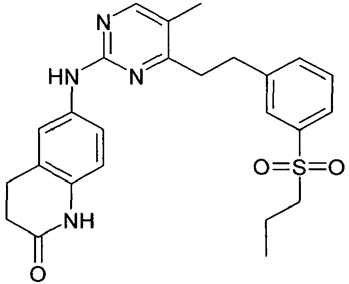 | |
| HPLC-MS; rt; [M+H⁺]* | | |
| RT: 3.33 min; | | |
| Masse gefunden [M]⁺: 465.2 | | |
| "A105" | 6-(5-Fluor-4-{2-[3-(propan-1-sulfonyl)-phenyl]-ethyl}-pyrimidin-2-ylamino)-3,4-dihydro-1H-chinolin-2-on | 8.14 (d, *J =* 1.5, 1H), 7.83 (s, 1H), 7.77 - 7.74 (m, 1H), 7.67 (s, 1H), 7.52 - 7.48 (m, 2H), 7.43 (s, 1H), 7.37 (dd, *J* = 8.4, 2.4, 1H), 7.06 (s, 1H), 6.72 (d, *J* = 8.4, 1H), 3.50 (s, 1H), 3.23 - 3.16 (m, 2H), 3.12 - 2.97 (m, 6H), 2.65 (dd, *J* = 8.4, 6.6, 2H), 1.78 - 1.66 (m, 2H), 0.99 (t, *J* = 7.4, 3H) |
| | 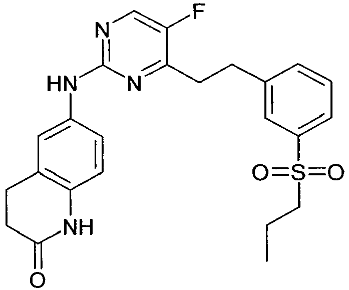 | |
| HPLC-MS; rt; [M+H⁺]* | | |
| RT: 4.10 min; | | |
| Masse gefunden [M]⁺: 469.3 | | |
| "A106" | [6-(4-tert.-Butyl-phenoxy)-pyridin-3-yl]-(5-fluor-4-phenethyl-pyrimidin-2-yl)-amin | 9.73 (s, 1H), 8.44 (d, *J* = 2.6, 1H), 8.39 (d, *J* = 1.3, 1H), 8.11 (dd, *J* = 8.8, 2.7, 1H), 7.39 (d, *J* = 8.6, 2H), 7.30 - 7.13 (m, 5H), 6.97 (t, *J* = 8.4, 3H), 3.01 (s, 4H), 1.28 (s, 9H) |
| | 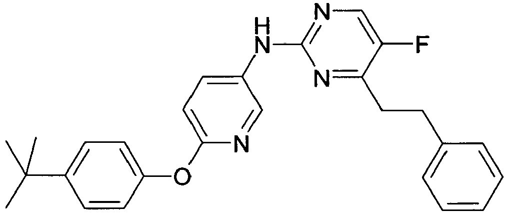 | |
| HPLC-MS; rt; [M+H⁺]* | | |
| RT: 6.68 min; | | |
| Masse gefunden [M]⁺: 443.2 | | |
| "A107" | (5-Fluor-4-phenethyl-pyrimidin-2-yl)-(6-trifluormethyl-pyridin-3-yl)-amin | 10.35 (s, 1H), 9.00 (d, *J* = 2.5, 1H), 8.52 (d, *J =* 1.8, 1H), 8.40 (dd, *J* = 8.6, 2.3, 1H), 7.81 (d, *J =* 8.7, 1H), 7.30 - 7.21 (m, 4H), 7.20 - 7.15 (m, 1H), 3.04 (m, 4H) |
| | 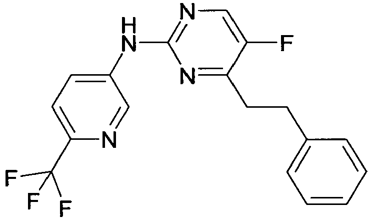 | |
| HPLC-MS; rt; [M+H⁺]* | | |
| RT: 5.65 min; | | |
| Masse gefunden [M]⁺: 363.3 | | |
| "A108" | (5-Fluor-4-phenethyl-pyrimidin-2-yl)-(6-fluor-pyridin-3-yl)-amin | 9.90 (s, 1H), 8.54 - 8.48 (m, 1H), 8.43 (d, *J* = 1.8, 1H), 8.21 (ddd, *J* = 8.9, 7.4, 2.9, 1H), 7.27 (dd, *J* = 10.0, 4.6, 2H), 7.24 - 7.14 (m, 3H), 7.11 (dd, *J* = 8.9, 3.3, 1H), 3.03 (s, 4H) |
| | 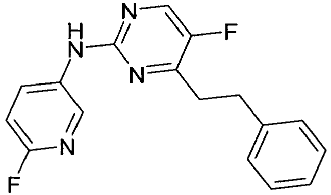 | |
| HPLC-MS; rt; [M+H⁺]* | | |
| RT: 5.05 min; | | |
| Masse gefunden [M]⁺: 313.3 | | |
| "A109" | 5-(5-Fluor-4-phenethyl-pyrimidin-2-ylamino)-pyridin-2-carbonitril | 10.50 (s, 1H), 8.96 (d, *J* = 2.4, 1H), 8.55 (d, *J* = 1.7, 1H), 8.37 (dd, *J* = 8.7, 2.6, 1H), 7.92 (d, *J* = 8.6, 1H), 7.22 (m, 5H), 3.06 (s, 4H) |
| | 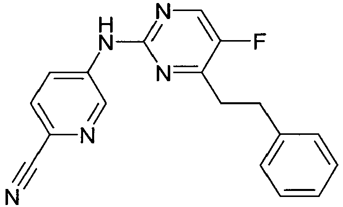 | |
| HPLC-MS; rt; [M+H⁺]* | | |
| RT: 5.01 min; | | |
| Masse gefunden [M]⁺: 320.3 | | |
| "A110" | N-[5-(5-Fluor-4-phenethyl-pyrimidin-2-ylamino)-pyridin-2-yl]-acetamid | 10.33 (s, 1H), 9.73 (s, 1H), 8.61 - 8.57 (m, 1H), 8.40 (d, *J* = 1.8, 1H), 8.04 (dd, *J* = 9.0, 2.7, 1H), 7.97 (d, *J* = 8.9, 1H), 7.31 - 7.14 (m, 5H), 3.02 (s, 4H), 2.07 (s, 3H) |
| | 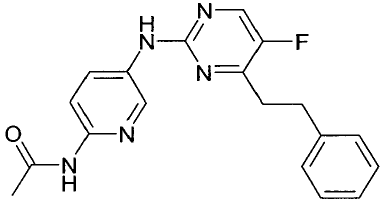 | |
| HPLC-MS; rt; [M+H⁺]* | | |
| RT: 3.75 min; | | |
| Masse gefunden [M]⁺: 352.3 | | |
| "A111" | N-Methyl-N-(2-{2-[2-(2-oxo-2,3-dihydro-1H-indol-5-ylamino)-5-trifluormethyl-pyrimidin-4-yl]-ethyl}-phenyl)-methansulfonamid | |
| | 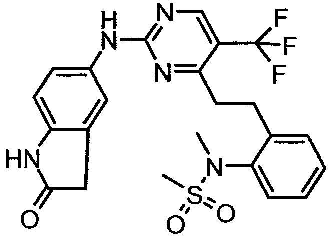 | |

Pharmakologische Testergebnisse

**Tabelle 1**

| FAK Inhibierung einiger erfindungsgemäßer Verbindungen der Formel I | | |
|---|---|---|
| No. | IC₅₀ (enzymatisch) | IC₅₀ (zellulär) |
| "A1" | C | |
| "A2" | A | C |
| "A3" | C | |
| "A5" | A | |
| "A6" | B | |
| "A7" | B | |
| "A9" | B | |
| "A11" | B | |
| "A13" | B | |
| "A16" | C | |
| "A20" | B | |
| "A21" | B | |
| "A22" | C | |
| "A24" | | C |
| "A25" | B | C |
| "A26" | | |
| "A27" | B | |
| "A28" | B | |
| "A29" | C | |
| "A30" | B | |
| "A31" | A | C |
| "A32" | B | |
| "A33" | B | |
| "A34" | A | B |
| "A35" | B | |
| "A36" | B | |
| "A37" | | B |
| "A38" | B | |
| "A39" | B | |
| "A40" | B | |
| "A41" | B | |
| "A43" | B | |
| "A44" | A | |
| "A45" | A | |
| "A46" | B | |
| | | |
| "A101" | B | |
| "A102" | B | |
| "A103" | B | |

| | | |
|---|---|---|
| IC₅₀: <0.3µM=A 0.3-3µM=B > 3-50 µM = C | | |

Die nachfolgenden Beispiele betreffen pharmazeutische Zubereitungen:

### Beispiel A: Injektionsgläser

Eine Lösung von 100 g eines Wirkstoffes der Formel I gemäß Anspruch 1 und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I gemäß Anspruch 1 mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I gemäß Anspruch 1, 9,38 g NaH₂PO₄ · 2 H₂O, 28,48 g Na₂HPO₄ · 12H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I gemäß Anspruch 1 mit 99,5 g Vaseline unter aseptischen Bedingungen.

### Beispiel E: Tabletten

Ein Gemisch von 1 kg Wirkstoff der Formel I gemäß Anspruch 1, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I gemäß Anspruch 1 werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I gemäß Anspruch 1 in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
| Nr. | Name und/oder Struktur |
|---|---|
| "A1" | N-(2-{2-[2-(4-Methansulfonyl-phenylamino)-pyrimidin-4-yl]-ethyl}-phenyl)-N-methyl-methansulfonamid |
| "A2" | N-Methyl-N-(2-{2-[2-(2-oxo-1,2,3,4-tetrahydro-chinolin-6-ylamino)-pyrimidin-4-yl]-ethyl}-phenyl)-methansulfonamid |
| "A3" | 6-{4-[2-(3-Methansulfonyl-phenyl)-ethyl]-pyrimidin-2-ylamino}-3,4-dihydro-1H-chinolin-2-on |
| "A4" | (4-Methansulfonyl-phenyl)-{4-[2-(3-methansulfonyl-phenyl)-ethyl]-pyrimidin-2-yl}-amin |
| "A5" | N-Methyl-N-(2-{2-[2-(2-oxo-2,3-dihydro-1H-indol-5-ylamino)-pyrimidin-4-yl]-ethyl}-phenyl)-methansulfonamid |
| "A6" | N-Methyl-N-(3-{2-[2-(2-oxo-2,3-dihydro-1H-indol-5-ylamino)-pyrimidin-4-yl]-ethyl}-pyridin-2-yl)-methansulfonamid |
| "A7" | N-Methyl-N-(3-{2-[2-(2-oxo-1,2,3,4-tetrahydro-chinolin-6-ylamino)-pyrimidin-4-yl]-ethyl}-pyridin-2-yl)-methansulfonamid |
| "A8" | N-(3-{2-[2-(4-Methansulfonyl-phenylamino)-pyrimidin-4-yl]-ethyl}-pyridin-2-yl)-N-methyl-methansulfonamid |
| "A9" | (2-{2-[2-(2-Oxo-2,3-dihydro-1H-indol-5-ylamino)-pyrimidin-4-yl]-ethyl}-phenyl)-acetonitril |
| "A10" | N-Methyl-N-(3-{2-[2-(2-oxo-2,3-dihydro-1H-indol-5-ylamino)-pyrimidin-4-yl]-ethyl}-pyridin-2-yl)-acetamid |
| "A11" | (4-Fluor-2-{2-[2-(2-oxo-2,3-dihydro-1H-indol-5-ylamino)-pyrimidin-4-yl]-ethyl}-phenyl)-acetonitril |
| "A12" | N-Methyl-N-(3-{2-[2-(2-oxo-2,3-dihydro-1H-indol-5-ylamino)-pyrimidin-4-yl]-ethyl}-pyridin-2-yl)-formamid |
| "A13" | 1-(2-{2-[2-(2-Oxo-2,3-dihydro-1H-indol-5-ylamino)-pyrimidin-4-yl]-ethyl}-phenyl)-cyclobutancarbonitril |
| "A14" | N-[2-(3-{2-[2-(2-Oxo-2,3-dihydro-1H-indol-5-ylamino)-pyrimidin-4-yl]-ethyl}-pyridin-2-ylamino)-ethyl]-formamid |
| "A15" | (2-{2-[2-(2-Oxo-2,3-dihydro-1H-indol-5-ylamino)-pyrimidin-4-yl]-ethyl}-5-trifluormethyl-phenyl)-acetonitril |
| "A16" | 3-(3-{2-[2-(2-Oxo-2,3-dihydro-1H-indol-5-ylamino)-pyrimidin-4-yl]-ethyl}-pyridin-2-yl)-azetidin-3-carbonitril |
| "A17" | (2-{2-[2-(2-Oxo-2,3-dihydro-1H-indol-5-ylamino)-pyrimidin-4-yl]-ethyl}-4-trifluormethyl-phenyl)-acetonitril |
| "A18" | (5-Fluor-2-{2-[2-(2-oxo-2,3-dihydro-1H-indol-5-ylamino)-pyrimidin-4-yl]-ethyl}-phenyl)-acetonitril |
| "A19" | 1-(2-{2-[2-(2-Oxo-2,3-dihydro-1H-indol-5-ylamino)-pyrimidin-4-yl]-ethyl}-4-trifluormethyl-phenyl)-cyclobutancarbonitril |
| "A20" | N-Methyl-N-(3-{2-[2-(2-oxo-2,3-dihydro-benzoxazol-6-ylamino)-pyrimidin-4-yl]-ethyl}-pyridin-2-yl)-methanesulfonamid |
| "A21" | N-Methyl-N-(3-{2-[2-(1-methyl-2-oxo-2,3-dihydro-1H-indol-5-ylamino)-pyrimidin-4-yl]-ethyl}-pyridin-2-yl)-methanesulfonamid |
| "A22" | N-(3-{2-[2-(3-Cyan-phenylamino)-pyrimidin-4-yl]-ethyl}-pyridin-2-yl)-N-methyl-methansulfonamid |
| "A23" | N-(3-{2-[2-(3-Cyan-4-fluor-phenylamino)-pyrimidin-4-yl]-ethyl}-pyridin-2-yl)-N-methyl-methansulfonamid |
| "A24" | N-Methyl-N-[3-(2-{2-[(6-oxo-1,6-dihydro-pyridazin-3-ylmethyl)-amino]-pyrimidin-4-yl}-ethyl)-pyridin-2-yl]-methansulfonamid |
| "A25" | N-(3-{2-[2-(1,3-Dimethyl-2-oxo-2,3-dihydro-1H-benzimidazol-5-ylamino)-pyrimidin-4-yl]-ethyl}-pyridin-2-yl)-N-methyl-methansulfonamid |
| "A26" | N-(3-{[5-Cyan-2-(2-oxo-2,3-dihydro-benzoxazol-6-ylamino)-pyrimidin-4-ylamino]-methyl}-pyridin-2-yl)-N-methyl-methansulfonamid |
| "A27" | N-Methyl-N-(2-{2-[2-(2-oxo-2,3-dihydro-benzoxazol-6-ylamino)-pyrimidin-4-yl]-ethyl}-phenyl)-methansulfonamid |
| "A28" | N-(2-{2-[2-(3-Cyan-phenylamino)-pyrimidin-4-yl]-ethyl}-phenyl)-N-methyl-methansulfonamid |
| "A29" | N-(2-{2-[2-(3-Cyan-4-fluor-phenylamino)-pyrimidin-4-yl]-ethyl}-phenyl)-N-methyl-methansulfonamid |
| "A30" | N-Methyl-N-(2-{2-[2-(2-oxo-2,3-dihydro-benzothiazol-5-ylamino)-pyrimidin-4-yl]-ethyl}-phenyl)-methansulfonamid |
| "A31" | N-(2-{2-[2-(1-Acetyl-2,3-dihydro-1H-indol-5-ylamino)-pyrimidin-4-yl]-ethyl}-phenyl)-N-methyl-methansulfonamid |
| "A32" | 2-[3-(4-{2-[2-(Methansulfonyl-methyl-amino)-phenyl]-ethyl}-pyrimidin-2-ylamino)-phenyl]-N-methyl-acetamid |
| "A33" | N-Methyl-N-[2-(2-{2-[4-(3-oxo-morpholin-4-yl)-phenylamino]-pyrimidin-4-yl}-ethyl)-phenyl]-methansulfonamid |
| "A34" | N-[4-(4-{2-[2-(Methansulfonyl-methyl-amino)-phenyl]-ethyl}-pyrimidin-2-ylamino)-phenyl]-nicotinamid |
| "A35" | N-Methyl-N-[2-(2-{2-[4-(2-oxo-pyrrolidin-1-yl)-phenylamino]-pyrimidin-4-yl}-ethyl)-phenyl]-methansulfonamid |
| "A36" | N-[2-(2-{2-[3,5-Dichlor-4-(2-oxo-pyrrolidin-1-yl)-phenylamino]-pyrimidin-4-yl}-ethyl)-phenyl]-N-methyl-methansulfonamid |
| "A37" | N-[2-(2-{2-[3-Cyan-4-(2-oxo-piperidin-1-yl)-phenylamino]-pyrimidin-4-yl}-ethyl)-phenyl]-N-methyl-methansulfonamid |
| "A38" | N-[2-(2-{2-[4-(1,3-Dioxo-1,3-dihydro-isoindol-2-yl)-phenylamino]-pyrimidin-4-yl}-ethyl)-phenyl]-N-methyl-methansulfonamid |
| "A39" | N-Methyl-N-[3-(2-{2-[4-(2-oxo-pyrrolidin-1-yl)-phenylamino]-pyrimidin-4-yl}-ethyl)-pyridin-2-yl]-methansulfonamid |
| "A40" | N-[4-(4-{2-[2-(Methansulfonyl-methyl-amino)-pyridin-3-yl]-ethyl}-pyrimidin-2-ylamino)-phenyl]-nicotinamid |
| "A41" | N-Methyl-N-[3-(2-{2-[4-(3-oxo-morpholin-4-yl)-phenylamino]-pyrimidin-4-yl}-ethyl)-pyridin-2-yl]-methansulfonamid |
| "A42" | N-[3-(2-{2-[3-Cyan-4-(2-oxo-piperidin-1-yl)-phenylamino]-pyrimidin-4-yl}-ethyl)-pyridin-2-yl]-N-methyl-methansulfonamid |
| "A43" | N-[3-(2-{2-[4-(1,3-Dioxo-1,3-dihydro-isoindol-2-yl)-phenylamino]-pyrimidin-4-yl}-ethyl)-pyridin-2-yl]-N-methyl-methansulfonamid |
| "A44" | N-Methyl-N-(5-methyl-3-{2-[2-(2-oxo-2,3-dihydro-1H-indol-5-ylamino)-pyrimidin-4-yl]-ethyl}-pyridin-2-yl)-methansulfonamid |
| "A45" | 1-(4-Fluor-2-{2-[2-(2-oxo-2,3-dihydro-1H-indol-5-ylamino)-pyrimidin-4-yl]-ethyl}-phenyl)-cyclobutancarbonitril |
| "A46" | 1-(5-Fluor-2-{2-[2-(2-oxo-2,3-dihydro-1H-indo)-5-ylamino)-pyrimidin-4-yl]-ethyl}-phenyl)-cyclobutancarbonitril |
| "A47" | 1-(2-{2-[2-(2-Oxo-2,3-dihydro-1H-indol-5-ylamino)-pyrimidin-4-yl]-ethyl}-5-trifluormethyl-phenyl)-cyclobutancarbonitril |
| "A48" | (5-Fluor-4-phenethyl-pyrimidin-2-yl)-phenyl-amin |
| "A49" | (5-Methyl-4-phenethyl-pyrimidin-2-yl)-phenyl-amin |
| "A50" | (4-Phenethyl-5-trifluormethyl-pyrimidin-2-yl)-phenyl-amin |
| "A52" | (5-Brom-4-phenethyl-pyrimidin-2-yl)-phenyl-amin |
| "A56" | 6-{5-Fluor-4-[2-(4-fluor-phenyl)-ethyl]-pyrimidin-2-ylamino}-3,4-dihydro-1H-chinolin-2-on |
| "A65" | 6-{4-[2-(4-fluor-phenyl)-ethyl]-5-methyl-pyrimidin-2-ylamino}-3,4-dihydro-1H-chinolin-2-on |
| "A71" | [5-(1-Methyl-1H-pyrazol-4-yl)-4-phenethyl-pyrimidin-2-yl]-phenyl-amin |
| "A74" | 5-{4-[2-(4-Fluor-phenyl)-ethyl]-5-trifluormethyl-pyrimidin-2-ylamino}-1,3-dihydro-indol-2-on |
| "A77" | 6-{4-[2-(4-Fluor-phenyl)-ethyl]-5-trifluoromethyl-pyrimidin-2-ylamino}-3,4-dihydro-1H-chinolin-2-on |
| "A101" | 6-(5-Brom-4-phenethyl-pyrimidin-2-ylamino)-3,4-dihydro-1H-chinolin-2-on |
| "A102" | 5-(4-Phenethyl-5-trifluormethyl-pyrimidin-2-ylamino)-1,3-dihydro-indol-2-on |
| "A103" | 6-(4-Phenethyl-5-trifluormethyl-pyrimidin-2-ylamino)-3,4-dihydro-1H-chinolin-2-on |
| "A104" | 6-(5-Methyl-4-{2-[3-(propan-1-sulfonyl)-phenyl]-ethyl}-pyrimidin-2-ylamino)-3,4-dihydro-1H-chinolin-2-on |
| "A105" | 6-(5-Fluor-4-{2-[3-(propan-1-sulfonyl)-phenyl]-ethyl}-pyrimidin-2-ylamino)-3,4-dihydro-1H-chinolin-2-on |
| "A106" | [6-(4-tert.-Butyl-phenoxy)-pyridin-3-yl]-(5-fluor-4-phenethyl-pyrimidin-2-yl)-amin |
| "A107" | (5-Fluor-4-phenethyl-pyrimidin-2-yl)-(6-trifluormethyl-pyridin-3-yl)-amin |
| "A108" | (5-Fluor-4-phenethyl-pyrimidin-2-yl)-(6-fluor-pyridin-3-yl)-amin |
| "A109" | 5-(5-Fluor-4-phenethyl-pyrimidin-2-ylamino)-pyridin-2-carbonitril |
| "A110" | N-[5-(5-Fluor-4-phenethyl-pyrimidin-2-ylamino)-pyridin-2-yl]-acetamid |
| "A111" | N-Methyl-N-(2-{2-[2-(2-oxo-2,3-dihydro-1H-indol-5-ylamino)-5-trifluormethyl-pyrimidin-4-yl]-ethyl}-phenyl)-methansulfonamid |
sowie ihre pharmazeutisch verwendbaren Salze, Tautomere und Stereoisomere, einschließlich deren Mischungen in allen Verhältnissen.

2. Arzneimittel, enthaltend mindestens eine Verbindung nach Anspruch 1 und/oder ihre pharmazeutisch verwendbaren Salze, Tautomeren und Stereoisomeren, einschließlich deren Mischungen in allen Verhältnissen, sowie gegebenenfalls Träger- und/oder Hilfsstoffe.

3. Verbindungen nach Anspruch 1 sowie ihrer pharmazeutisch verwendbaren Salze, Tautomeren und Stereoisomeren, einschließlich deren Mischungen in allen Verhältnissen,
zur Verwendung zur Behandlung von Tumoren, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, Autoimmunerkrankungen, Zirrhose, Diabetes und Erkrankungen der Blutgefässe.

## Claims

1. Compounds selected from the group
| No. | Name and/or structure |
|---|---|
| "A1" | N-(2-{2-[2-(4-Methanesulfonylphenylamino)pyrimidin-4-yl]ethyl}phenyl)-N-methylmethanesulfonamide |
| "A2" | N-Methyl-N-(2-{2-[2-(2-oxo-1,2,3,4-tetrahydroquinolin-6-ylamino)pyrimidin-4-yl]ethyl}phenyl)methanesulfonamide |
| "A3" | 6-{4-[2-(3-Methanesulfonylphenyl)ethyl]pyrimidin-2-yl-amino}-3,4-dihydro-1H-quinolin-2-one |
| "A4" | (4-Methanesulfonylphenyl)-{4-[2-(3-methanesulfonylphenyl)ethyl]pyrimidin-2-yl}amine |
| "A5" | N-Methyl-N-(2-{2-[2-(2-oxo-2,3-dihydro-1H-indol-5-yl-amino)pyrimidin-4-yl]ethyl}phenyl)methanesulfonamide |
| "A6" | N-Methyl-N-(3-{2-[2-(2-oxo-2,3-dihydro-1H-indol-5-yl-amino)pyrimidin-4-yl]ethyl}pyridin-2-yl)methane-sulfonamide |
| "A7" | N-Methyl-N-(3-{2-[2-(2-oxo-1,2,3,4-tetrahydroquinolin-6-yl-amino)pyrimidin-4-yl]ethyl}pyridin-2-yl)methanesulfonamide |
| "A8" | N-(3-{2-[2-(4-Methanesulfonylphenylamino)pyrimidin-4-yl]ethyl}pyridin-2-yl)-N-methylmethanesulfonamide |
| "A9" | (2-{2-[2-(2-Oxo-2,3-dihydro-1H-indol-5-ylamino)pyrimidin-4-yl]ethyl}phenyl)acetonitrile |
| "A10" | N-Methyl-N-(3-{2-[2-(2-oxo-2,3-dihydro-1H-indol-5-yl-amino)pyrimidin-4-yl]ethyl}pyridin-2-yl)acetamide |
| "A11" | (4-Fluoro-2-{2-[2-(2-oxo-2,3-dihydro-1H-indol-5-ylamino)-pyrimidin-4-yl]ethyl}phenyl)acetonitrile |
| "A12" | N-Methyl-N-(3-{2-[2-(2-oxo-2,3-dihydro-1H-indol-5-yl-amino)pyrimidin-4-yl]ethyl}pyridin-2-yl)formamide |
| "A13" | 1-(2-{2-[2-(2-Oxo-2,3-dihydro-1H-indol-5-ylamino)-pyrimidin-4-yl]ethyl}phenyl)cyclobutanecarbonitrile |
| "A14" | N-[2-(3-{2-[2-(2-Oxo-2,3-dihydro-1H-indol-5-ylamino)-pyrimidin-4-yl]ethyl}pyridin-2-ylamino)ethyl]formamide |
| "A15" | (2-{2-[2-(2-Oxo-2,3-dihydro-1H-indol-5-ylamino)pyrimidin-4-yl]ethyl}-5-trifluoromethylphenyl)acetonitrile |
| "A16" | 3-(3-{2-[2-(2-Oxo-2,3-dihydro-1H-indol-5-ylamino)-pyrimidin-4-yl]ethyl}pyridin-2-yl)azetidine-3-carbonitrile |
| "A17" | (2-{2-[2-(2-Oxo-2,3-dihydro-1H-indol-5-ylamino)pyrimidin-4-yl]ethyl}-4-trifluoromethylphenyl)acetonitrile |
| "A18" | (5-Fluoro-2-{2-[2-(2-oxo-2,3-dihydro-1H-indol-5-ylamino)-pyrimidin-4-yl]ethyl}phenyl)acetonitrile |
| "A19" | 1-(2-{2-[2-(2-Oxo-2,3-dihydro-1H-indol-5-ylamino)-pyrimidin-4-yl]ethyl}-4-trifluoromethylphenyl)-cyclobutanecarbonitrile |
| "A20" | N-Methyl-N-(3-{2-[2-(2-oxo-2,3-dihydrobenzoxazol-6-yl-amino)pyrimidin-4-yl]ethyl}pyridin-2-yl)-methanesulfonamide |
| "A21" | N-Methyl-N-(3-{2-[2-(1-methyl-2-oxo-2,3-dihydro-1H-indol-5-ylamino)pyrimidin-4-yl]ethyl}pyridin-2-yl)-methanesulfonamide |
| "A22" | N-(3-{2-[2-(3-Cyanophenylamino)pyrimidin-4-yl]ethyl}-pyridin-2-yl)-N-methylmethanesulfonamide |
| "A23" | N-(3-{2-[2-(3-Cyano-4-fluorophenylamino)pyrimidin-4-yl]-ethyl}pyridin-2-yl)-N-methylmethanesulfonamide |
| "A24" | N-Methyl-N-[3-(2-{2-[(6-oxo-1,6-dihydropyridazin-3-yl-methyl)amino]pyrimidin-4-yl}ethyl)pyridin-2-yl]-methanesulfonamide |
| "A25" | N-(3-{2-[2-(1,3-Dimethyl-2-oxo-2,3-dihydro-1H-benzimidazol-5-ylamino)pyrimidin-4-yl]ethyl}pyridin-2-yl)-N-methylmethanesulfonamide |
| "A26" | N-(3-{[5-Cyano-2-(2-oxo-2,3-dihydrobenzoxazol-6-yl-amino)pyrimidin-4-ylamino]methyl}pyridin-2-yl)-N-methylmethanesulfonamide |
| "A27" | N-Methyl-N-(2-{2-[2-(2-oxo-2,3-dihydrobenzoxazol-6-yl-amino)pyrimidin-4-yl]ethyl}phenyl)methanesulfonamide |
| "A28" | N-(2-{2-[2-(3-Cyanophenylamino)pyrimidin-4-yl]ethyl}-phenyl)-N-methylmethanesulfonamide |
| "A29" | N-(2-{2-[2-(3-Cyano-4-fluorophenylamino)pyrimidin-4-yl]-ethyl}phenyl)-N-methylmethanesulfonamide |
| "A30" | N-Methyl-N-(2-{2-[2-(2-oxo-2,3-dihydrobenzothiazol-5-yl-amino)pyrimidin-4-yl]ethyl}phenyl)methanesulfonamide |
| "A31" | N-(2-{2-[2-(1-Acetyl-2,3-dihydro-1H-indol-5-ylamino)-pyrimidin-4-yl]ethyl}phenyl)-N-methylmethanesulfonamide |
| "A32" | 2-[3-(4-{2-[2-(Methanesulfonylmethylamino)phenyl]ethyl}-pyrimidin-2-ylamino)phenyl]-N-methylacetamide |
| "A33" | N-Methyl-N-[2-(2-{2-[4-(3-oxomorpholin-4-yl)phenyl-amino]pyrimidin-4-yl}ethyl)phenyl]methanesulfonamide |
| "A34" | N-[4-(4-{2-[2-(Methanesulfonylmethylamino)phenyl]ethyl}-pyrimidin-2-ylamino)phenyl]nicotinamide |
| "A35" | N-Methyl-N-[2-(2-{2-[4-(2-oxopyrrolidin-1-yl)phenylamino]-pyrimidin-4-yl}ethyl)phenyl]methanesulfonamide |
| "A36" | N-[2-(2-{2-[3,5-Dichloro-4-(2-oxopyrrolidin-1-yl)phenyl-amino]pyrimidin-4-yl}ethyl)phenyl]-N-methylmethanesulfonamide |
| "A37" | N-[2-(2-{2-[3-Cyano-4-(2-oxopiperidin-1-yl)phenylamino]-pyrimidin-4-yl}ethyl)phenyl]-N-methylmethanesulfonamide |
| "A38" | N-[2-(2-{2-[4-(1,3-Dioxo-1,3-dihydroisoindol-2-yl)phenyl-amino]pyrimidin-4-yl}ethyl)phenyl]-N-methylmethanesulfonamide |
| "A39" | N-Methyl-N-[3-(2-{2-[4-(2-oxopyrrolidin-1-yl)phenylamino]-pyrimidin-4-yl}ethyl)pyridin-2-yl]methanesulfonamide |
| "A40" | N-[4-(4-{2-[2-(Methanesulfonylmethylamino)pyridin-3-yl]-ethyl}pyrimidin-2-ylamino)phenyl]nicotinamide |
| "A41" | N-Methyl-N-[3-(2-{2-[4-(3-oxomorpholin-4-yl)phenyl-amino]pyrimidin-4-yl}ethyl)pyridin-2-yl]methanesulfonamide |
| "A42" | N-[3-(2-{2-[3-Cyano-4-(2-oxopiperidin-1-yl)phenylamino]-pyrimidin-4-yl}ethyl)pyridin-2-yl]-N-methylmethanesulfonamide |
| "A43" | N-[3-(2-{2-[4-(1,3-Dioxo-1,3-dihydroisoindol-2-yl)phenyl-amino]pyrimidin-4-yl}ethyl)pyridin-2-yl]-N-methylmethanesulfonamide |
| "A44" | N-Methyl-N-(5-methyl-3-{2-[2-(2-oxo-2,3-dihydro-1H-indol-5-ylamino)pyrimidin-4-yl]ethyl}pyridin-2-yl)-methanesulfonamide |
| "A45" | 1-(4-Fluoro-2-{2-[2-(2-oxo-2,3-dihydro-1H-indol-5-yl-amino)pyrimidin-4-yl]ethyl}phenyl)cyclobutanecarbonitrile |
| "A46" | 1-(5-Fluoro-2-{2-[2-(2-oxo-2,3-dihydro-1H-indol-5-yl-amino)pyrimidin-4-yl]ethyl}phenyl)cyclobutanecarbonitrile |
| "A47" | 1-(2-{2-[2-(2-Oxo-2,3-dihydro-1H-indol-5-ylamino)-pyrimidin-4-yl]ethyl}-5-trifluoromethylphenyl)-cyclobutanecarbonitrile |
| "A48" | (5-Fluoro-4-phenethylpyrimidin-2-yl)phenylamine |
| "A49" | (5-Methyl-4-phenethylpyrimidin-2-yl)phenylamine |
| "A50" | (4-Phenethyl-5-trifluoromethylpyrimidin-2-yl)phenylamine |
| "A52" | (5-Bromo-4-phenethylpyrimidin-2-yl)phenylamine |
| "A56" | 6-{5-Fluoro-4-[2-(4-fluorophenyl)ethyl]pyrimidin-2-yl-amino}-3,4-dihydro-1H-quinolin-2-one |
| "A65" | 6-{4-[2-(4-Fluorophenyl)ethyl]-5-methylpyrimidin-2-yl-amino}-3,4-dihydro-1H-quinolin-2-one |
| "A71" | [5-(1-Methyl-1H-pyrazol-4-yl)-4-phenethylpyrimidin-2-yl]-phenylamine |
| "A74" | 5-{4-[2-(4-Fluorophenyl)ethyl]-5-trifluoromethylpyrimidin-2-ylamino}-1,3-dihydroindol-2-one |
| "A77" | 6-{4-[2-(4-Fluorophenyl)ethyl]-5-trifluoromethylpyrimidin-2-ylamino}-3,4-dihydro-1H-quinolin-2-one |
| "A101" | 6-(5-Bromo-4-phenethylpyrimidin-2-ylamino)-3,4-dihydro-1H-quinolin-2-one |
| "A102" | 5-(4-Phenethyl-5-trifluoromethylpyrimidin-2-ylamino)-1,3-dihydroindol-2-one |
| "A103" | 6-(4-Phenethyl-5-trifluoromethylpyrimidin-2-ylamino)-3,4-dihydro-1H-quinolin-2-one |
| "A104" | 6-(5-Methyl-4-{2-[3-(propane-1-sulfonyl)phenyl]ethyl}pyrimidin-2-ylamino)-3,4-dihydro-1H-quinolin-2-one |
| "A105" | 6-(5-Fluoro-4-{2-[3-(propane-1-sulfonyl)phenyl]ethyl}pyrimidin-2-ylamino)-3,4-dihydro-1H-quinolin-2-one |
| "A106" | [6-(4-tert-Butylphenoxy)pyridin-3-yl]-(5-fluoro-4-phenethylpyrimidin-2-yl)amine |
| "A107" | (5-Fluoro-4-phenethylpyrimidin-2-yl)-(6-trifluoromethylpyridin-3-yl)amine |
| "A108" | (5-Fluoro-4-phenethylpyrimidin-2-yl)-(6-fluoropyridin-3-yl)amine |
| "A109" | 5-(5-Fluoro-4-phenethylpyrimidin-2-ylamino)pyridine-2-carbonitrile |
| "A110" | N-[5-(5-Fluoro-4-phenethylpyrimidin-2-ylamino)pyridin-2-yl]-acetamide |
| "A111" | N-Methyl-N-(2-{2-[2-(2-oxo-2,3-dihydro-1H-indol-5-yl-amino)-5-trifluoromethylpyrimidin-4-yl]ethyl}phenyl)-methanesulfonamide |
and pharmaceutically usable salts, tautomers and stereoisomers thereof, including mixtures thereof in all ratios.

2. Medicaments comprising at least one compound according to Claim 1 and/or pharmaceutically usable salts, tautomers and stereoisomers thereof, including mixtures thereof in all ratios, and optionally excipients and/or adjuvants.

3. Compounds according to Claim 1 and pharmaceutically usable salts, tautomers and stereoisomers thereof, including mixtures thereof in all ratios,
for use for the treatment of tumours, cancer, tumour formation, growth and spread, arteriosclerosis, eye diseases, such as age-induced macular degeneration, choroidal neovascularisation and diabetic retinopathy, inflammatory diseases, arthritis, thrombosis, fibrosis, glomerulonephritis, neurodegeneration, psoriasis, restenosis, wound healing, transplant rejection, metabolic diseases and diseases of the immune system, autoimmune diseases, cirrhosis, diabetes and diseases of the blood vessels.

## Revendications

1. Composés choisis dans le groupe constitué par
| n° | Nom et/ou structure |
|---|---|
| « A1 » | N-(2-{2-[2-(4-Méthanesulfonylphénylamino)pyrimidin-4-yl]éthyl}phényl)-N-méthylméthanesulfonamide |
| « A2 » | N-Méthyl-N-(2-{2-[2-(2-oxo-1,2,3,4-tétrahydroquinoléin-6-ylamino)pyrimidin-4-yl]éthyl}phényl)méthanesulfonamide |
| « A3 » | 6-{4-[2-(3-Méthanesulfonylphényl)éthyl]pyrimidin-2-yl-amino}-3,4-dihydro-1H-quinoléin-2-one |
| « A4 » | (4-Méthanesulfonylphényl)-{4-[2-(3-méthanesulfonylphényl)éthyl]pyrimidin-2-yl}amine |
| « A5 » | N-Méthyl-N-(2-{2-[2-(2-oxo-2,3-dihydro-1H-indol-5-yl-amino)pyrimidin-4-yl]éthyl}phényl)méthanesulfonamide |
| « A6 » | N-Méthyl-N-(3-{2-[2-(2-oxo-2,3-dihydro-1H-indol-5-yl-amino)pyrimidin-4-yl]éthyl}pyridin-2-yl)méthanesulfonamide |
| « A7 » | N-Méthyl-N-(3-{2-[2-(2-oxo-1,2,3,4-tétrahydroquinoléin-6-ylamino)pyrimidin-4-yl]éthyl}pyridin-2-yl)méthanesulfonamide |
| « A8 » | N-(3-{2-[2-(4-Méthanesulfonylphénylamino)pyrimidin-4-yl]éthyl}pyridin-2-yl)-N-méthylméthanesulfonamide |
| « A9 » | (2-{2-[2-(2-Oxo-2,3-dihydro-1H-indol-5-ylamino)-pyrimidin-4-yl]éthyl}phényl)acétonitrile |
| « A10 » | N-Méthyl-N-(3-{2-[2-(2-oxo-2,3-dihydro-1H-indol-5-yl-amino)pyrimidin-4-yl]éthyl}pyridin-2-yl)acétamide |
| « A11 » | (4-Fluoro-2-{2-[2-(2-oxo-2,3-dihydro-1H-indol-5-yl-amino)pyrimidin-4-yl]éthyl}phényl)acétonitrile |
| « A12 » | N-Méthyl-N-(3-{2-[2-(2-oxo-2,3-dihydro-1H-indol-5-yl-amino)pyrimidin-4-yl]éthyl}pyridin-2-yl)formamide |
| « A13 » | 1-(2-{2-[2-(2-Oxo-2,3-dihydro-1H-indol-5-ylamino)-pyrimidin-4-yl]éthyl}phényl)cyclobutanecarbonitrile |
| « A14 » | N-[2-(3-{2-[2-(2-Oxo-2,3-dihydro-1H-indol-5-yl-amino)pyrimidin-4-yl]éthyl}pyridin-2-ylamino)éthyl]-formamide |
| « A15 » | (2-{2-[2-(2-Oxo-2,3-dihydro-1H-indol-5-ylamino)-pyrimidin-4-yl]éthyl}-5-trifluorométhylphényl)acétonitrile |
| « A16 » | 3-(3-{2-[2-(2-Oxo-2,3-dihydro-1H-indol-5-ylamino)-pyrimidin-4-yl]éthyl}pyridin-2-yl)azétidine-3-carbonitrile |
| « A17 » | (2-{2-[2-(2-Oxo-2,3-dihydro-1H-indol-5-ylamino)-pyrimidin-4-yl]éthyl}-4-trifluorométhylphényl)acétonitrile |
| « A18 » | (5-Fluoro-2-{2-[2-(2-oxo-2,3-dihydro-1H-indol-5-yl-amino)pyrimidin-4-yl]éthyl}phényl)acétonitrile |
| « A19 » | 1-(2-{2-[2-(2-Oxo-2,3-dihydro-1H-indol-5-ylamino)-pyrimidin-4-yl]éthyl}-4-trifluorométhylphényl)-cyclobutanecarbonitrile |
| « A20 » | N-Méthyl-N-(3-{2-[2-(2-oxo-2,3-dihydrobenzoxazol-6-yl-amino)pyrimidin-4-yl]éthyl}pyridin-2-yl)méthanesulfonamide |
| « A21 » | N-Méthyl-N-(3-{2-[2-(1-méthyl-2-oxo-2,3-dihydro-1H-indol-5-ylamino)pyrimidin-4-yl]éthyl}pyridin-2-yl)-méthanesulfonamide |
| « A22 » | N-(3-{2-[2-(3-Cyanophénylamino)pyrimidin-4-yl]éthyl}pyridin-2-yl)-N-méthylméthanesulfonamide |
| « A23 » | N-(3-{2-[2-(3-Cyano-4-fluorophénylamino)pyrimidin-4-yl]-éthyl}pyridin-2-yl)-N-méthylméthanesulfonamide |
| « A24 » | N-Méthyl-N-[3-(2-{2-[(6-oxo-1,6-dihydropyridazin-3-yl-méthyl)amino]pyrimidin-4-yl}éthyl)pyridin-2-yl]-méthanesulfonamide |
| « A25 » | N-(3-{2-[2-(1,3-Diméthyl-2-oxo-2,3-dihydro-1H-benzimidazol-5-ylamino)pyrimidin-4-yl]éthyl}pyridin-2-yl)-N-méthylméthanesulfonamide |
| « A26 » | N-(3-{[5-Cyano-2-(2-oxo-2,3-dihydrobenzoxazol-6-yl-amino)pyrimidin-4-ylamino]méthyl}pyridin-2-yl)-N-méthylméthanesulfonamide |
| « A27 » | N-Méthyl-N-(2-{2-[2-(2-oxo-2,3-dihydrobenzoxazol-6-yl-amino)pyrimidin-4-yl]éthyl}phényl)méthanesulfonamide |
| « A28 » | N-(2-{2-[2-(3-Cyanophénylamino)pyrimidin-4-yl]éthyl}phényl)-N-méthylméthanesulfonamide |
| « A29 » | N-(2-{2-[2-(3-Cyano-4-fluorophénylamino)pyrimidin-4-yl]éthyl}phényl)-N-méthylméthanesulfonamide |
| « A30 » | N-Méthyl-N-(2-{2-[2-(2-oxo-2,3-dihydrobenzothiazol-5-yl-amino)pyrimidin-4-yl]éthyl}phényl)méthanesulfonamide |
| « A31 » | N-(2-{2-[2-(1-Acétyl-2,3-dihydro-1H-indol-5-ylamino)-pyrimidin-4-yl]éthyl}phényl)-N-méthylméthanesulfonamide |
| « A32 » | 2-[3-(4-{2-[2-(Méthanesulfonylméthylamino)phényl]-éthyl}pyrimidin-2-ylamino)phényl]-N-méthylacétamide |
| « A33 » | N-Méthyl-N-[2-(2-{2-[4-(3-oxomorpholin-4-yl)phényl-amino]pyrimidin-4-yl}éthyl)phényl]méthanesulfonamide |
| « A34 » | N-[4-(4-{2-[2-(Méthanesulfonylméthylamino)phényl]-éthyl}pyrimidin-2-ylamino)phényl]nicotinamide |
| « A35 » | N-Méthyl-N-[2-(2-{2-[4-(2-oxopyrrolidin-1-yl)phényl-amino]pyrimidin-4-yl}éthyl)phényl]méthanesulfonamide |
| « A36 » | N-[2-(2-{2-[3,5-Dichloro-4-(2-oxopyrrolidin-1-yl)phényl-amino]pyrimidin-4-yl}éthyl)phényl]-N-méthylméthanesulfonamide |
| « A37 » | N-[2-(2-{2-[3-Cyano-4-(2-oxopipéridin-1-yl)phényl-amino]pyrimidin-4-yl}éthyl)phényl]-N-méthylméthanesulfonamide |
| « A38 » | N-[2-(2-{2-[4-(1,3-Dioxo-1,3-dihydroisoindol-2-yl)phényl-amino]pyrimidin-4-yl}éthyl)phényl]-N-méthylméthanesulfonamide |
| « A39 » | N-Méthyl-N-[3-(2-{2-[4-(2-oxopyrrolidin-1-yl)phényl-amino]pyrimidin-4-yl}éthyl)pyridin-2-yl]méthane-sulfonamide |
| « A40 » | N-[4-(4-{2-[2-(Méthanesulfonylméthylamino)pyridin-3-yl]-éthyl}pyrimidin-2-ylamino)phényl]nicotinamide |
| « A41 » | N-Méthyl-N-[3-(2-{2-[4-(3-oxomorpholin-4-yl)phényl-amino]pyrimidin-4-yl}éthyl)pyridin-2-yl]méthanesulfonamide |
| « A42 » | N-[3-(2-{2-[3-Cyano-4-(2-oxopipéridin-1-yl)phényl-amino]pyrimidin-4-yl}éthyl)pyridin-2-yl]-N-méthylméthanesulfonamide |
| « A43 » | N-[3-(2-{2-[4-(1,3-Dioxo-1,3-dihydroisoindol-2-yl)phényl-amino]pyrimidin-4-yl}éthyl)pyridin-2-yl]-N-méthylméthanesulfonamide |
| « A44 » | N-Méthyl-N-(5-méthyl-3-{2-[2-(2-oxo-2,3-dihydro-1H-indol-5-ylamino)pyrimidin-4-yl]éthyl}pyridin-2-yl)-méthanesulfonamide |
| « A45 » | 1-(4-Fluoro-2-{2-[2-(2-oxo-2,3-dihydro-1H-indol-5-yl-amino)pyrimidin-4-yl]éthyl}phényl)cyclobutane-carbonitrile |
| « A46 » | 1-(5-Fluoro-2-{2-[2-(2-oxo-2,3-dihydro-1H-indol-5-yl-amino)pyrimidin-4-yl]éthyl}phényl)cyclobutane-carbonitrile |
| « A47 » | 1-(2-{2-[2-(2-Oxo-2,3-dihydro-1H-indol-5-ylamino)-pyrimidin-4-yl]éthyl}-5-trifluorométhylphényl)-cyclobutanecarbonitrile |
| « A48 » | (5-Fluoro-4-phénéthylpyrimidin-2-yl)phénylamine |
| « A49 » | (5-Méthyl-4-phénéthylpyrimidin-2-yl)phénylamine |
| « A50 » | (4-Phénéthyl-5-trifluorométhylpyrimidin-2-yl)-phénylamine |
| « A52 » | (5-Bromo-4-phénéthylpyrimid in-2-yl)phénylamine |
| « A56 » | 6-{5-Fluoro-4-[2-(4-fluorophényl)éthyl]pyrimidin-2-yl-amino}-3,4-dihydro-1H-quinoléin-2-one |
| « A65 » | 6-{4-[2-(4-Fluorophényl)éthyl]-5-méthylpyrimidin-2-yl-amino}-3,4-dihydro-1H-quinoléin-2-one |
| « A71 » | [5-(1-Méthyl-1H-pyrazol-4-yl)-4-phénéthylpyrimidin-2-yl]-phénylamine |
| « A74 » | 5-{4-[2-(4-Fluorophényl)éthyl]-5-trifluorométhylpyrimidin-2-ylamino}-1,3-dihydroindol-2-one |
| « A77 » | 6-{4-[2-(4-Fluorophényl)éthyl]-5-trifluorométhylpyrimidin-2-ylamino}-3,4-dihydro-1H-quinoléin-2-one |
| « A101 » | 6-(5-Bromo-4-phénéthylpyrimidin-2-ylamino)-3,4-dihydro-1H-quinoléin-2-one |
| « A102 » | 5-(4-Phénéthyl-5-trifluorométhylpyrimidin-2-ylamino)-1,3-dihydroindol-2-one |
| « A103 » | 6-(4-Phénéthyl-5-trifluorométhylpyrimidin-2-ylamino)-3,4-dihydro-1H-quinoléin-2-one |
| « A104 » | 6-(5-Méthyl-4-{2-[3-(propane-1-sulfonyl)phényl]éthyl}-pyrimidin-2-ylamino)-3,4-dihydro-1H-quinoléin-2-one |
| « A105 » | 6-(5-Fluoro-4-{2-[3-(propane-1-sulfonyl)phényl]éthyl}-pyrimidin-2-ylamino)-3,4-dihydro-1H-quinoléin-2-one |
| « A106 » | [6-(4-tertio-Butylphénoxy)pyridin-3-yl]-(5-fluoro-4-phénéthylpyrimidin-2-yl)amine |
| « A107 » | (5-Fluoro-4-phénéthylpyrimidin-2-yl)-(6-trifluorométhyl-pyridin-3-yl)amine |
| « A108 » | (5-Fluoro-4-phénéthylpyrimidin-2-yl)-(6-fluoropyridin-3-yl)amine |
| « A109 » | 5-(5-Fluoro-4-phénéthylpyrimidin-2-ylamino)pyridine-2-carbonitrile |
| « A110 » | N-[5-(5-Fluoro-4-phénéthylpyrimidin-2-ylamino)pyridin-2-yl]acétamide |
| « A111 » | N-Méthyl-N-(2-{2-[2-(2-oxo-2,3-dihydro-1H-indol-5-yl-amino)-5-trifluorométhylpyrimidin-4-yl]éthyl}phényl)-méthanesulfonamide |
ainsi que les sels, tautomères et stéréoisomères pharmaceutiquement utilisables de ceux-ci, y compris des mélanges de ceux-ci en toutes proportions.

2. Médicaments comprenant au moins un composé selon la revendication 1, et/ou des sels, tautomères et stéréoisomères pharmaceutiquement utilisables de celui-ci, y compris des mélanges de ceux-ci en toutes proportions, et éventuellement des excipients et/ou des adjuvants.

3. Composés selon la revendication 1 et les sels, tautomères et stéréoisomères pharmaceutiquement utilisables de ceux-ci, y compris des mélanges de ceux-ci en toutes proportions,
pour une utilisation dans le traitement de tumeurs, du cancer, de la formation, de la croissance et de la propagation de tumeurs, de l'artériosclérose, de maladies oculaires, telles que la dégénérescence maculaire liée à l'âge, la néovascularisation choroïdienne et la rétinopathie diabétique, des maladies inflammatoires, de l'arthrite, de la thrombose, de la fibrose, de la glomérulonéphrite, de la neurodégénérescence, du psoriasis, de la resténose, de la guérison de plaies, du rejet de greffe, des maladies métaboliques et des maladies du système immunitaire, des maladies auto-immunes, de la cirrhose, du diabète et des maladies des vaisseaux sanguins.
